# EUROPEAN PATENT APPLICATION

(11) **EP 1 099 442 A2**
(43) Date of publication of application: **16.05.2001**
(21) Application number: 00309907.4
(22) Date of filing: 08.11.2000
(51) Int. Cl.: A61K 31/472, A61K 31/4725, A61K 31/47, A61K 31/4709, A61K 31/4468, A61K 31/4985, A61K 31/437, A61P 3/06

(54) **Methods of administering apo B-secretion/MTP inhibitors**

(30) Priority: 10.11.1999 US 164579 P
(71) Applicant: Pfizer Products Inc., Groton, Connecticut 06340 (US)
(72) Inventor: Chang, George, Pfizer Central Research, Groton, Connecticut 06340 (US); Vincent, John, Pfizer Central Research, Groton, Connecticut 06340 (US)
(74) Representative: Ruddock, Keith Stephen

(57) **Abstract**

The invention provides methods for administering of apolipoprotein B-secretion (Apo B)/microsomal triglyceride transfer protein (MTP) inhibitors which comprise administering the inhibitor to a subject in need of treatment therewith prior to, or during, a period of somnolence.

## Description

### BACKGROUND OF THE INVENTION

The invention relates to improved methods of reducing levels of total serum cholesterol and LDL-cholesterol in a subject in need of such reduction and to methods of administering apolipoprotein B (apo B) secretion/microsomal triglyceride transfer protein (MTP) inhibitors to a subject in need of treatment therewith.

Microsomal triglyceride transfer protein catalyzes the transport of triglyceride, cholesteryl ester and phospolipids and has been strongly implicated as a mediator in the assembly of apo B-containing lipoproteins, biomolecules which contribute to the formation of atherosclerotic lesions. Specifically, the subcellular (lumen of the endoplasmic reticulum) and tissue distribution (liver and intestine) of MTP have led to speculation that it plays a role in the assembly of plasma lipoproteins, as these are known sites of plasma lipoprotein assembly. The ability of MTP to catalyze the transport of triglyceride between membranes is consistent with this speculation and suggests that MTP may catalyze the transport of triglyceride from its site of synthesis in the endoplasmic reticulum membrane to nascent lipoprotein particles within the lumen of the endoplasmic reticulum.

Compounds that inhibit apo B-secretion and/or inhibit MTP are accordingly useful in the treatment of diseases and conditions in which, by inhibiting apo B-secretion and/or MTP, serum cholesterol and triglyceride levels may be reduced. Such conditions may include, for example, hypercholesterolemia, hypertriglyceridemia, pancreatitis, atherosclerosis, diabetes and the like. For detailed discussions see, for example, Wetterau et al., Science, 258, 999-1001 (1992) and Wetterau et al., Biochem. Biophys. Acta., 875, 610-617 (1986).

Specific examples of compounds having utility as apo B-secretion/MTP inhibitors are disclosed in European Patent Application Publication Nos. EP 0 584 446 and EP 0 643 057, the latter of which discloses certain compounds of the generic formulae 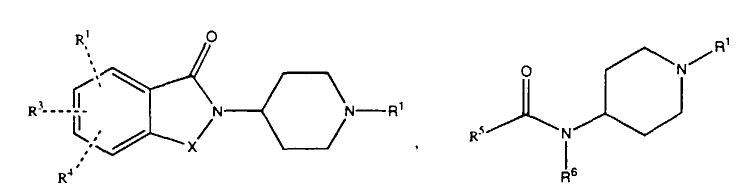 and 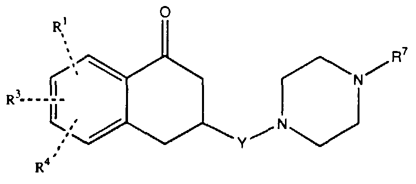 which have utility as inhibitors of MTP.

Furthermore, commonly assigned PCT International Application Publication Nos. WO 96/40640 and WO 98/23593, each of which designate, *inter alia,* the United States, disclose certain tetrahydroisoquinolines useful as apo B secretion/MTP inhibitors. The disclosures of the aforementioned PCT International Application Publication Nos. WO 96/40640 and WO 98/23593 are incorporated herein by reference. Additional apo B secretion/MTP inhibitors useful in the practice of the instant invention are known, or will be apparent in light of this disclosure, to one of ordinary skill in the art.

In studies assessing the impact of various circadian dosing regimens on the efficacy of hypercholesterolemic drugs, only competitive inhibitors of 3-hydroxy-3-methylglutaryl-coenzyme A reductase (HMG-CoA reductase) have been investigated. For example, two studies, involving the HMG-CoA reductase inhibitors mevinolin and pravastatin respectively, have indicated some slight improvement in the reduction of cholesterol levels can be effected when the drugs are administered in the evening as compared to the morning. However, the overall influence of evening as opposed to morning administration on cholesterol levels was marginal at best, resulting only in decreases of between 3 and 7%. See Illingworth, Clin. Pharmacol. Ther., 40, 338-343 (1986) and Hunninghake, et al., 85, 219-227 (1990). In another study employing the HMG-CoA reductase inhibitor simvastatin, the overall reduction of serum cholesterol levels compared to placebo was also minimal and changes in triglyceride and HDL-cholesterol levels were not significantly different from placebo. See Saito, et al., Arteriosclerosis and Thrombosis, 11, 816-826 (1991). A more recent study of the HMG-CoA reductase inhibitor atorvastatin demonstrated no significant decrease in total cholesterol, LDL-cholesterol, or apolipoprotein levels compared to placebo when the drug was administered in the evening rather than in the morning. See Cilla, et al., J. Clin. Pharmacol., 36, 604-609 (1996).

In direct contrast to the above results, it has now been found that a substantial reduction of total serum cholesterol and LDL-cholesterol levels can be achieved by administering an apo B-secretion/MTP inhibitor, to a subject in need of treatment therewith, prior to, or during, a somnolent period of the subject being treated.

### SUMMARY OF THE INVENTION

The invention provides methods of reducing total cholesterol and LDL-cholesterol, which methods comprise administering to a subject in need of such reduction an effective amount of an apolipoprotein B-secretion (apo B)/microsomal triglyceride transfer protein (MTP) inhibitor prior to, or during, a somnolent period of the subject being treated.

The invention further provides methods of administering apolipoprotein B-secretion (apo B)/microsomal triglyceride transfer protein (MTP) inhibitors to a subject in need of treatment therewith which methods comprise administering an effective amount of the inhibitor prior to, or during, a somnolent period of the subject being treated.

The apo B-secretion/MTP inhibitor, as employed according to the methods of the instant invention, is preferably selected from:
(i) a compound of formula (I) 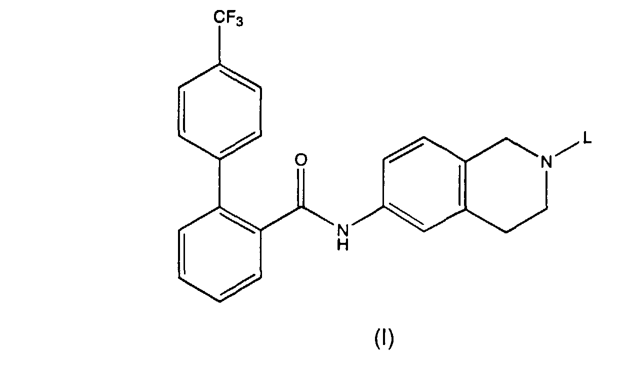 the stereoisomers and hydrates thereof, and the pharmaceutically acceptable salts of said compounds, stereoisomers and hydrates, wherein L is as defined hereinbelow;
(ii) a compound of formula (Ia) 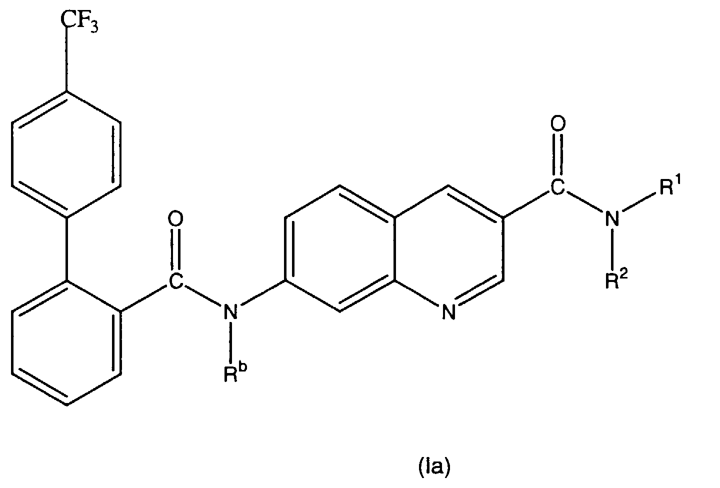 the stereoisomers and prodrugs thereof, and the pharmaceutically acceptable salts of the compounds, stereoisomers, and prodrugs, wherein R¹, R²,and R^{b} are as defined hereinbelow; and
(iii) a compound selected from the group consisting of:
   9-[4-[4-(2,3-dihydro-1-oxo-1H-isoindol-2-yl)-1-piperidinyl]butyl]-N-propyl-9H-fluorene-9-carboxamide;
   2-[1-(3,3-diphenylpropyl)-4-piperidinyl]-2,3-dihydro-1H-isoindol-1-one;
   9-[4-(4-[2-(4-trifluoromethylphenyl)benzoylamino]piperidin-1-yl)butyl]-N-2,2,2-trifluoroethyl)-9H-fluorene-9-carboxamide;
   9-{4-[4-(2-benzothiazol-2-yl-benzoylamino)-piperidin-1-yl]-butyl}-9H-fluorene-9-carboxylic acid-(2,2,2-trifluoroethyl)-amide;
   [11a-R]-8-[(4-cyanophenyl)methoxy]-2-cyclopentyl-7-(prop-2-enyl)-2,3,11,11a-tetrahydro-6H-pyrazino[1,2b]isoquinoline-1,4-dione;
   [11a-R]-cyclopentyl-7-(prop-2-enyl)-8-[(pyridin-2-yl)methoxy]-2,3,11,11a-tetrahydro-6H-pyrazino[1,2b]-isoquinoline-1,4-dione;
   2-cyclopentyl-2-[4-(2,4-dimethyl-pyrido[2,3b]indol-9-ylmethyl)-phenyl]-N-(2-hydroxy-1-phenyl-ethyl)-acetamide; and
   2-cyclopentyl-N-(2-hydroxy-1-phenyl-ethyl)-2-[4-(quinolin-2-ylmethoxy)-phenyl]-acetamide; and the pharmaceutically acceptable salts thereof.

### DETAILED DESCRIPTION OF THE INVENTION

The invention provides methods of reducing total serum cholesterol and LDL-cholesterol, which methods comprise administering to a subject in need of such reduction an effective amount of an apolipoprotein B-secretion (apo B)/microsomal triglyceride transfer protein (MTP) inhibitor prior to, or during, a somnolent period of the subject being treated.

The invention further provides methods of administering apolipoprotein B-secretion (apo B)/microsomal triglyceride transfer protein (MTP) inhibitors to a subject in need of treatment therewith which methods comprise administering the inhibitor prior to, or during, a somnolent period of the subject being treated.

As employed throughout the instant description and appendant claims, the phrase "somnolent period" refers generally to the normal sleeping period of the subject being treated. Preferably, the apo B-secretion/MTP inhibitor is administered to the subject just prior to the normal evening sleeping event (e.g. at bedtime). It is to be specifically understood, however, that the somnolent period may also take place during daylight hours where required by the normal sleeping schedule and/or the psychological predisposition or predilection of the subject.

Although any apo B-secretion/MTP inhibitor may be employed in the methods of the instant invention, it is generally preferred that the inhibitor be selected from:
(i) a compound of formula (I) 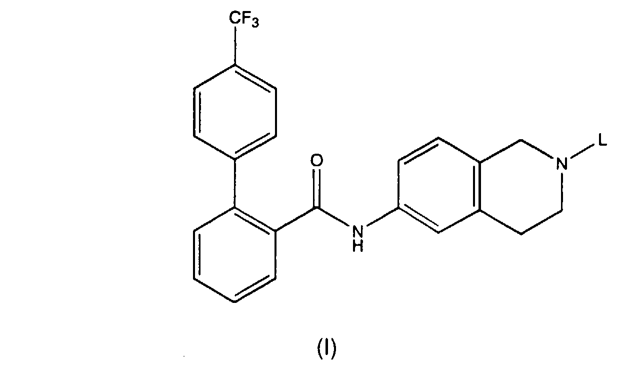 the stereoisomers and hydrates thereof, and the pharmaceutically acceptable salts of the compounds, stereoisomers and hydrates, wherein L represents:
X-Y-Z, wherein:
X is a moiety selected from the group consisting of CH₂, CO, CS, or SO₂;
Y is a moiety selected from the group consisting of a direct link, aliphatic hydrocarbylene radicals having up to 20 carbon atoms, which radical may be monosubstituted by hydroxy, (C₁-C₁₀)alkoxy, (C₁-C₁₀)acyl, (C₁-C₁₀)acyloxy, or (C₆-C₁₀)aryl, NH, and O, provided that if X is CH₂, Y is a direct link; and
Z is a moiety selected from the group consisting of:
   (1) hydrogen, halogen, cyano,
   (2) hydroxy, (C₁-C₁₀)alkoxy, (C₁-C₁₀)alkylthio, (C₁-C₁₀)acyl, thiophenylcarbonyl, (C₁-C₁₀)alkoxycarbonyl,
   (3) (C₁-C₁₀)alkylamino, di(C₁-C₁₀)alkylamino, (C₆-C₁₀)aryl(C₁-C₁₀)alkylamino, provided that Y is not O or NH,
   (4) unsubstituted vinyl, (C₆-C₁₀)aryl, (C₃-C₈)cycloalkyl and fused benz derivatives thereof, (C₇-C₁₀)polycycloalkyl, (C₄-C₈)cycloalkenyl, (C₇-C₁₀)polycycloalkenyl,
   (5) (C₆-C₁₀)aryloxy, (C₆-C₁₀)arylthio, (C₆-C₁₀)aryl(C₁-C₁₀)alkoxy, (C₆-C₁₀)aryl(C₁-C₁₀)alkylthio, (C₃-C₈)cycloalkyloxy, (C₄-C₈)cycloalkenyloxy,
   (6) heterocyclyl selected from the group consisting of monocyclic radicals and fused polycyclic radicals, wherein said radicals contain a total of from 5 to 14 ring atoms, wherein said radicals contain a total of from 1 to 4 ring heteroatoms independently selected from oxygen, nitrogen, and sulfur, and wherein the individual rings of said radicals may be independently saturated, partially unsaturated, or aromatic, provided that if X is CH₂, Z is H or is selected from groups (4) and (6),
      wherein, when Z contains one or more rings, said rings may each independently bear 0 to 4 substituents independently selected from halo, hydroxy, cyano, nitro, oxo, thioxo, aminosulfonyl, phenyl, phenoxy, phenylthio, halophenylthio, benzyl, benzyloxy, (C₁-C₁₀)alkyl, (C₁-C₁₀)alkoxy, (C₁-C₁₀)alkoxycarbonyl, (C₁-C₁₀)alkylthio, (C₁-C₁₀)alkylamino, (C₁-C₁₀)alkylaminocarbonyl, di(C₁-C₁₀)alkylamino, di(C₁-C₁₀)alkylaminocarbonyl, di(C₁-C₁₀)alkylamino(C₁-C₁₀)alkoxy, (C₁-C₃)perfluoroalkyl, (C₁-C₃)perfluoroalkoxy, (C₁-C₁₀)acyl, (C₁-C₁₀)acyloxy, (C₁-C₁₀)acyloxy(C₁-C₁₀)alkyl, and pyrrolidinyl; or
G, wherein G is selected from the group consisting of:
   (a) a phenyl or heterocyclic ring wherein said heterocyclic ring contains a total of from 3 to 14 ring atoms, wherein said heterocyclic ring incorporates a total of from 1 to 4 ring heteroatoms selected independently from oxygen, nitrogen, and sulfur, wherein the individual rings of said heterocyclic ring may be independently saturated, partially saturated or aromatic, and wherein each of said phenyl or heterocyclic rings may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acyloxy, (C₁-C₆)acylamino and (C₁-C₆)perfluoroacylamino;
   (b) -CH₂CN,
   (c) 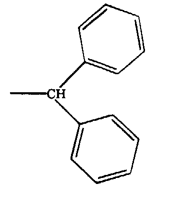
   (d) (C₂-C₁₂)alkyl or (C₂-C₁₂)perfluoroalkyl wherein each of said (C₂-C₁₂)alkyl and (C₂-C₁₂)perfluoroalkyl is substituted optionally with from 1-3 substituents selected independently from:
      (1) phenyl, halogen, nitro, cyano, hydroxy, -NR¹R², -OCOR³, (C₁-C₄)alkoxy, (C₁-C₄)perfluoroalkoxy, (C₁-C₄)thioalkoxy or (C₁-C₄)perfluorothioalkoxy,
         where R¹ and R² in the definition of -NR¹R² are each selected independently from hydrogen, formyl, phenyl, benzyl, benzoyl, (C₃-C₈)cycloalkyl, (C₃-C₈)cycloalkenyl, (C₁-C₄)alkyl, (C₁-C₄)perfluoroalkyl, (C₁-C₁₀)alkoxycarbonyl, (C₁-C₆)acyl, (C₁-C₆)perfluoroacyl, aminocarbonyl, (C₁-C₁₀)alkylaminocarbonyl, di(C₁-C₁₀)alkylaminocarbonyl, aminosulfonyl, (C₁-C₄)alkylaminosulfonyl, di(C₁-C₄)alkylaminosulfonyl, (C₁-C₄)perfluoroalkylaminosulfonyl, di(C₁-C₄)perfluoroalkylaminosulfonyl, (C₁-C₄)alkylsulfonyl, and (C₁-C₄)perfluoroalkylsulfonyl,
         or where R¹ and R², taken together with the nitrogen atom to which they are attached, form a saturated, partially-saturated or aromatic heterocyclic ring, wherein said heterocyclic ring contains a total of from 3 to 14 ring atoms and incorporates optionally an additional 1 to 4 ring heteroatoms selected independently from oxygen, nitrogen and sulfur, wherein said heterocyclic ring may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-p₁₀)Perfluoroacyl, (C₁-C₁₀)acylamino, and (C₁-C₁₀)acyloxy,
         where R³ in the definition of -OCOR³ is selected from -NR¹R², phenyl, (C₁-C₁₀)alkyl, (C₁-C₄)perfluoroalkyl, (C₁-C₆)alkoxy and (C₁-C₆)perfluoroalkoxy,
      (2) (C₃-C₈)cycloalkyl or (C₃-C₈)cycloalkenyl wherein each of said (C₃-C₈)cycloalkyl and (C₃-C₈)cycloalkenyl may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acylamino, (C₁-C₁₀)perfluoroacylamino, (C₁-C₁₀)acyloxy, and
      (3) a saturated, partially-saturated or aromatic heterocyclic ring containing a total of from 3 to 14 ring atoms, wherein said heterocyclic ring incorporates a total of from 1 to 4 ring heteroatoms selected independently from oxygen, nitrogen and sulfur, wherein said heterocyclic ring may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acylamino, (C₁-C₁₀)perfluoroacylamino, (C₁-C₁₀)acyloxy;
   (e) (C₃-C₈)cycloalkyl or (C₃-C₈)cycloalkenyl wherein each of said (C₃-C₈)cycloalkyl and (C₃-C₈)cycloalkenyl may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acylamino, (C₁-C₁₀)perfluoroacylamino, (C₁-C₁₀)acyloxy; and
   (f) -(CH₂)ₙCOR⁴, where R⁴ in the definition of -(CH₂)ₙCOR⁴ is selected from hydroxy, phenyl, -NR¹R², (C₁-C₄)alkyl, (C₁-C₄)perfluoroalkyl, (C₁-C₄)alkoxy, (C₁-C₄)perfluoroalkoxy, (C₃-C₈)cycloalkyl, and (C₃-C₈)cycloalkenyl, where n is an integer from 1 to 4;
(ii) a compound of formula (Ia) 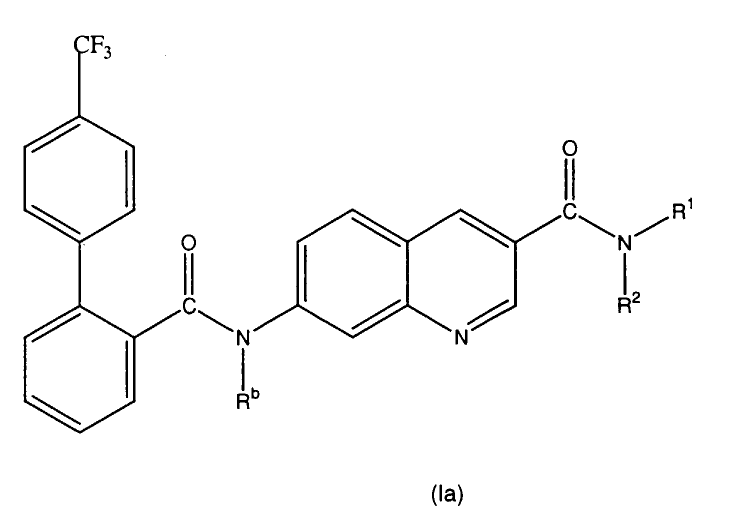 the stereoisomers and prodrugs thereof, and the pharmaceutically acceptable salts of the compounds, stereoisomers, and prodrugs, wherein
   each R^{a} and R^{b} is independently hydrogen or C₁-C₈alkyl;
   each n is independently 0, 1, 2 or 3;
   each X is independently aryl, substituted aryl, heteroaryl, substituted heteroaryl,
   cycloalkyl, substituted cycloalkyl, heterocycloalkyl, or substitited heterocycloalkyl;
   R¹ is hydrogen or C₁-C₈alkyl; and
   R² is hydrogen, -(CR^{a}R^{a})ₙ-X, C₁-C₈alkyl, C₁-C₈ substituted alkyl, 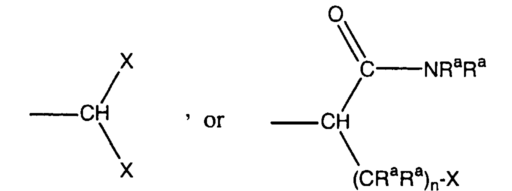 or R¹ and R² together with the nitrogen atom to which they are bonded form a 3 to 7 membered heterocycloalkyl ring comprising from 1 to 3 heteroatoms; and
(iii) a compound selected from the group consisting of:
   9-[4-[4-(2,3-dihydro-1-oxo-1H-isoindol-2-yl)-1-piperidinyl)butyl]-N-propyl-9H-fluorene-9-carboxamide;
   2-[1-(3,3-diphenylpropyl)-4-piperidinyl]-2,3-dihydro-1H-isoindol-1-one;
   9-[4-(4-[2-(4-trifluoromethylphenyl)benzoylamino]piperidin-1-yl)butyl]-N-2,2,2-trifluoroethyl)-9H-fluorene-9-carboxamide;
   9-{4-[4-(2-benzothiazol-2-yl-benzoylamino)-piperidin-1-yl]-butyl}-9H-fluorene-9-carboxylic acid-(2,2,2-trifluoroethyl)-amide;
   [11a-R]-8-[(4--cyanophenyl)methoxy]-2-cyclopentyl-7-(prop-2-enyl)-2,3,11,11a-tetrahydro-6H-pyrazino[1,2b]isoquinoline-1,4-dione;
   [11a-R]-cyclopentyl-7-(prop-2-enyl)-8-[(pyridin-2-yl)methoxy]-2,3,11,11atetrahydro-6H-pyrazino[1,2b]-isoquinoline-1,4-dione;
   2-cyclopentyl-2-[4-(2,4-dimethyl-pyrido[2,3b]indol-9-ylmethyl)-phenyl]-N-(2-hydroxy-1-phenyl-ethyl)-acetamide; and
   2-cyclopentyl-N-(2-hydroxy-1-phenyl-ethyl)-2-[4-(quinolin-2-ylmethoxy)-phenyl]-acetamide; and the pharmaceutically acceptable salts thereof.

A preferred subgroup of formula (I) compounds are those compounds selected from the group consisting of:
4'-trifluoromethyl-biphenyl-2-carboxylic acid-(2-(1H-(1,2,4-triazol-3-ylmethyl)-1,2,3,4-tetrahydroisoquinolin-6-yl)-amide;
4'-trifluoromethyl-biphenyl-2-carboxylic acid-[2-(2-acetylaminoethyl)-1,2,3,4-tetrahydroisoquinolin-6-yl amide;
4'-trifluoromethyl-biphenyl-2-carboxylic acid-[2-(2-methoxyethyl)-1,2,3,4-tetrahydroisoquinolin-6-yl]-amide;
4'-trifluoromethyl-biphenyl-2-carboxylic acid-[2-(2,2-diphenylethyl)-1,2,3,4-tetrahydroisoquinolin-6-yl]-amide;
2-{6-[4'-trifluoromethyl-biphenyl-2-carbonyl)-amino]-3,4-dihydro-1H-isoquinolin-2-yl}-ethyl)-carbamic acid methyl ester;
4'-trifluoromethyl-biphenyl-2-carboxylic acid-[2-(thiophen-2-yl-acetyl)-1,2,3,4-tetrahydroisoquinolin-6-yl]-amide;
4'-trifluoromethyl-biphenyl-2-carboxylic acid-(2-pyridin-2-ylmethyl)-1,2,3,4-tetrahydroisoquinolin-6-yl)-amide; and
6-[(4'-trifluoromethyl-biphenyl-2-carbonyl)-amino]-3,4-dihydro-1H-isoquinoline-2-carboxylic acid [(R)-1-phenyl-ethyl]-amide; the stereoisomers and hydrates thereof, and the pharmaceutically acceptable salts of the compounds, stereoisomers, and hydrates.

Unless noted otherwise, the definitions and illustrative values set forth hereinbelow for certain substituents and functional groups pertain both to compounds of formula (I) and (la).

The term "heterocyclyl" as employed within the definition of Z in the compounds of formula (I) is meant to embrace any single ring or fused ring system containing at least one ring heteroatom independently selected from O, N, and S. Thus, a polycyclic fused ring system containing one or more carbocyclic fused saturated, partially unsaturated or aromatic rings (usually benz rings) is within the definition of heterocyclyl so long as the system also contains at least one fused ring which contains at least one of the aforementioned heteroatoms. As a substituent, such heterocyclyls may be attached to the remainder of the molecule from either a carbocyclic (e.g. benz) ring or from a heterocyclic ring.

The phrase "one or more rings" when employed in the definition of Z is intended to mean any (single or fused) cyclic moiety or moieties contained in Z. The rings may be carbocyclic or heterocyclic, saturated or partially unsaturated and aromatic or non-aromatic.

Reference to a fused polycyclic ring system or radical means that all rings in the system are fused.

The term "acyl" when employed in the description of a substituent refers to an aliphatic or hydrocarbon moiety attached to a carbonyl group through which the substituent bonds. Representative of such acyl moieties are acetyl, propionyl, butyryl, isobutyryl, and the like.

The term "alkoxy" means an alkyl group bonded to an oxygen atom. Representative examples of alkoxy groups include methoxy, ethoxy, tert-butoxy, propoxy, and isobutoxy. Preferred alkoxy groups in formula (la) compounds are C₁-C₈alkoxy.

The term "alkyl" means a straight or branched chain hydrocarbon. Representative examples of alkyl groups include methyl, ethyl, propyl, isopropyl, butyl, isobutyl, tert-butyl, sec-butyl, pentyl, and hexyl. Preferred alkyl groups in formula (la) compounds are C₁-C₈alkyl. It is to be understood that references to individual radicals, for example "propyl" or "propoxy", embrace only the straight chain radical, branched chain isomers such as "isopropyl" or "isopropoxy" being referred to specifically.

The term "aliphatic hydrocarbylene radical" in reference to formula (I) compounds means a divalent, open-chain organic radical containing carbon and hydrogen only. The radical serves as a linking group, denoted hereinabove as Y. The radical may be straight chain or branched and/or saturated or unsaturated, containing up to three unsaturated bonds, either double, triple or a mixture of double and triple. The two valences may be on different carbon atoms or on the same carbon atom, and thus the term "alkylidene" is subsumed under this definition. The radical will typically be classified as a (C₁-C₂₀)alkylene radical, a (C₂-C₂₀)alkenylene radical or a (C₂-C₂₀)alkynylene radical. Typically, the radical will contain 1-10 carbon atoms, although longer chains are certainly feasible and within the scope of this invention.

The term "aryl", e.g. (C₆-C₁₀)aryl, when employed in the description of a substituent means the ring or substituent is carbocyclic. Aromatic moieties which contain one or more heteroatoms are included as a subset of of the term "heterocyclyl" as discussed hereinabove.

The term "cycloalkyl" means a cyclic hydrocarbon. Examples of cycloalkyl groups include cyclopropyl, cyclobutyl, cyclopentyl, cyclohexyl and cycloheptyl. Preferred cycloalkyl groups are C₃-C₈cyloalkyl. It is also possible for the cycloalkyl group to incorporate one or more double bonds or triple bonds, or a combination of double bonds and triple bonds, but not be aromatic. Examples of cycloalkyl groups having a double or triple bond include cyclopentenyl, cyclohexenyl, cyclohexadienyl, cyclobutadienyl, and the like.

The term "halogen" as employed throughout the description and appendant claims is inclusive of fluoro, chloro, bromo and iodo, unless noted otherwise.

The term "heteroaryl" as employed in formula (la) compounds means a cyclic, aromatic hydrocarbon in which one or more carbon atoms have been replaced with a heteroatom. If the heteroaryl group contains more than one heteroatom, the heteroatoms may be the same or different. Examples of heteroaryl groups include pyridyl, pyrimidinyl, imidazolyl, thienyl, furyl, pyrazinyl, pyrrolyl, pyranyl, isobenzofuranyl, chromenyl, xanthenyl, indolyl, isoindolyl, indolizinyl, triazolyl, pyridazinyl, indazolyl, purinyl, quinolizinyl, isoquinolyl, quinolyl, phthalazinyl, naphthyridinyl, quinoxalinyl, isothiazolyl, and benzo[b]thienyl. Preferred heteroaryl groups are five- and six-membered rings and contain from one to three heteroatoms. Further exemplary heteroaryl groups are illustrated in greater detail hereinbelow.

The term "heterocycloalkyl" in formula (la) compounds denotes a cycloalkyl group in which one or more of the carbon atoms has been replaced with a heteroatom. If the heterocycloalkyl group contains more than one heteroatom, the heteroatoms may be the same, or different. Examples of heterocycloalkyl groups include tetrahydrofuryl, morpholinyl, piperazinyl, piperadyl, and pyrrolidinyl. Preferred heterocycloalkyl groups are five- and six-membered rings and contain from one to three heteroatoms. It is also possible for the heterocycloalkyl group to have one or more double bonds or triple bonds or a combination of double bonds and triple bonds, but is not aromatic. Examples of heterocycloalkyl groups containing double or triple bonds include dihydrofuran, and the like.

The term "perfluoro", when employed in conjunction with a specified hydrocarbon radical, is meant to include a substituent wherein the individual hydrogen atoms thereof may be substituted therefor with one or more, and preferably, from 1 to 9 fluorine atoms. Exemplary of such radicals are trifluoromethyl, pentafluoroethyl, heptafluoropropyl, and the like.

The central benz-heterocyclic ring system of formula (I), i.e. the fused bicyclic ring system attached through its single ring nitrogen to L, is referred to herein as a "1,2,3,4-tetrahydroisoquinoline" for convenience in nomenclature, and this is the convention most commonly employed when naming compounds according to the invention as 2-substituted-1,2,3,4-tetrahydroisoquinolin-6-yl amides. It is noted that, less frequently, when named as a substituent in a compound, this central ring system is also denoted as a 6-substituted "3,4-dihydro-1H-isoquinolin-2-yl" moiety.

It will be appreciated by one of ordinary skill in the art that certain formula (I) and (la) compounds may contain an asymmetrically substituted carbon atom and accordingly may exist in, and be isolated in, both optically-active and racemic forms. Some compounds may exhibit polymorphism. It is to be understood that the instant invention encompasses any racemic, optically-active, polymorphic, stereoisomeric, or mixture thereof, forms of formula (I) and (la) compounds which forms possess properties useful in the methods of this invention. It is well known, or will be apparent in light of the instant disclosure, to one of ordinary skill in the art how to prepare such optically-active forms (for example, by resolution of the racemic form by recrystallization techniques, by synthesis from optically-active starting materials, by chiral synthesis or by chromatographic techniques) and how to determine the efficacy of such forms in carrying out the objectives of the present invention by the standard protocols described in detail hereinbelow.

Furthermore, one of ordinary skill in the art will recognize that certain combinations of substituents or moieties listed in this invention define compounds that may be less stable under physiological conditions (e.g., those compounds containing aminal or acetal linkages). Accordingly, such compounds are less preferred.

Alkylene radicals include those saturated hydrocarbon groups having 1-20, preferably 1-10 carbon atoms, derived by removing two hydrogen atoms from a corresponding saturated acyclic hydrocarbon. Illustrative values having 1-10 carbon atoms include straight chain radicals having the formula (CH₂)ₙ wherein n is 1 to 10, such as methylene, dimethylene, trimethylene, tetramethylene, pentamethylene, hexamethylene, heptamethylene, octamethylene nonamethylene and so forth. Also included are alkylidene radicals such as ethylidene, propylidene, butylidene and *sec*-butylidene. Also included are branched isomers such as 1,1-dimethyldimethylene, 1,1-dimethyltetramethylene, 2,2-dimethyltrimethylene and 3,3-dimethylpentamethylene.

Alkenylene radicals include those straight or branched chain radicals having 2-20 carbon atoms, preferably 2-10 carbon atoms, derived by removal of two hydrogen atoms from a corresponding acyclic hydrocarbon group containing at least one double bond. Illustrative values for alkenylene radicals having one double bond include ethenylene (vinylene), propenylene, 1-butenylene, 2-butenylene and isobutenylene. Alkenylene radicals containing two double bonds (sometimes referred to in the art as alkadienylene radicals) include 3-methyl-2,6-heptadienylene, 2-methyl-2,4-heptadienylene, 2,8-nonadienylene, 3-methyl-2,6-octadienylene and 2,6-decadienylene. An illustrative value for an alkylene radical containing three double bonds (an alkatrienylene radical) is 9,11,13-heptadecatrienylene.

Alkynylene radicals include those straight or branched chain radicals having 2-20 carbon atoms, preferably 2-10 carbon atoms, derived by removal of two hydrogen atoms from a corresponding acylic hydrocarbon group containing at least one triple bond. Illustrative values include ethynylene, propynylene, 1-butynylene, 1-pentynylene, 1-hexynylene, 2-butynylene, 2-pentynylene, 3,3-dimethyl-1-butynylene and so forth.

The following are exemplary values for certain moieties and substituents named hereinabove for compounds of formula (I) and (la), which are not to be construed as limiting in any respect. It is noted that throughout the instant description and appendant claims, if a cyclic or polycyclic radical which can be bonded through differing ring atoms is referred to without noting a specific point of attachment, all possible points are intended, whether through a carbon atom, or a trivalent nitrogen atom. For example, reference to (unsubstituted) "naphthyl" means naphth-1-yl, and naphth-2-yl; reference to "pyridyl" means 2-, 3-, or 4-pyridyl and reference to "indolyl" means attachment or bonding through any of the 1-, 2-, 3-, 4-, 5-, 6- or 7-positions.

Illustrative values for (C₁-C₁₀)alkoxy include methoxy, ethoxy, propoxy, isopropoxy, butoxy, isobutoxy, pentoxy, hexoxy, heptoxy and so forth.

Illustrative values for (C₁-C₁₀)alkylthio include the corresponding sulfur-containing compounds of (C₁-C₁₀)alkoxy hereinabove including methylthio, ethylthio, propylthio, isopropylthio, butylthio, isobutylthio, pentylthio, hexylthio, heptylthio and so forth.

Illustrative values for (C₁-C₁₀)acyl include values for (C₁-C₁₀)alkanoyl such as formyl, acetyl, propionyl, butyryl and isobutyryl. Also included are other common cycle-containing radicals such as benzoyl.

Illustrative values for (C₁-C₁₀)acyloxy include values for (C₁-C₁₀)alkanoyloxy such as formyloxy, acetyloxy, propionyloxy, butyryloxy and isobutyryloxy. Also included are other cycle-containing radicals such as benzoyloxy.

Illustrative values for (C₁-C₁₀)alkoxycarbonyl include methoxycarbonyl, ethoxycarbonyl, propoxycarbonyl, isopropoxycarbonyl, butoxycarbonyl and isobutoxycarbonyl.

Illustrative values for (C₁-C₁₀)alkylamino include methylamino, ethylamino, propylamino, isopropylamino, butylamino and isobutylamino.

Illustrative values for di-(C₁-C₁₀)alkylamino include dimethylamino, diethylamino, dipropylamino, dibutylamino and diisobutylamino.

Illustrative values for (C₆-C₁₀)aryl(C₁-C₁₀)alkylamino are benzylamino, (1-phenylethyl)amino and (2-phenylethyl)amino.

Illustrative values for (C₆-C₁₀)aryl include phenyl and naphthyl.

Illustrative values of (C₃-C₈)cycloalkyl include cyclopropyl, cyclobutyl, cyclcopentyl, cyclohexyl and cycloheptyl.

Illustrative values for fused benz derivatives of (C₃-C₈)cycloalkyl include 1,2,3,4-tetrahydronapthalenyl, indanyl and fluorenyl.

Illustrative values of polycycloalkyl include adamantyl and 2-bicyclo[2.2.1 ]heptyl.

Illustrative values for (C₄-C₈)cycloalkenyl include cyclobutenyl, cyclopentenyl, cyclohexenyl and cycloheptenyl.

Illustrative values for polycycloalkenyl include bicyclo[3.1.1]hept-2-enyl.

Illustrative values for (C₆-C₁₀)aryloxy include phenoxy and naphthyloxy.

Illustrative values for (C₆-C₁₀)arylthio include phenylthio and naphthylthio.

Illustrative values for (C₆-C₁₀)aryl(C₁-C₁₀)alkoxy include benzyloxy and phenylethoxy.

Illustrative values for (C₆-C₁₀)aryl(C₁-C₁₀)alkylthio include benzylthio and phenylethylthio.

Illustrative values for (C₃-C₈)cycloalkyloxy include cyclopropyloxy, cyclobutyloxy, cyclopentyloxy, cyclohexyloxy and cycloheptyloxy.

Illustrative values for (C₄-C₈)cycloalkenyloxy include cyclobutenyloxy, cyclopentenyloxy, cyclohexenyloxy and cycloheptenyloxy.

Illustrative values for heterocyclyl substituents which are five-membered monocyclic radicals include 1,3-dioxolanyl, 1,2-dithiolyl, 1,3-dithiolyl, 3H-1,2,3-dioxazolyl, 1,2,4-dioxazolyl, 1,3,4-dioxazolyl, 5H-1,2,5-oxathiazolyl, furanyl, thienyl, pyrrolyl, pyrrolidinyl, oxazolyl, 1,2,3-oxadiazolyl, 1,2,4-oxadiazolyl, 1,2,5-oxadiazolyl, 1,3,4-oxadiazolyl, 1,3-oxathiolyl, thiazolyl, imidazolyl, pyrazolyl, pyrazolidinyl, isoxazolyl, isothiazolyl, 1,2,3-triazolyl, 1,2,4-triazolyl, 1,3,4-thiadiazolyl, and the like.

Illustrative values for heterocyclyl substituents which are six-membered monocyclic radicals include 1,2-dioxinyl, 1,3-dioxinyl, 1,4-dioxanyl, 1,4-dithianyl, 2H-and 4H-pyranyl, pyridyl, piperidinyl, piperazinyl, pyridazinyl, pyrimidinyl, pyrazinyl, morpholinyl, thiomorpholinyl, 1,2,4-triazinyl, 1,2,3-triazinyl, 1,3,5-triazinyl, 1,3,5-trithianyl, 4H-1,2-oxazinyl, 2H-1,3-oxazinyl, 6H-1,3-oxazinyl, 6H-1,2-oxazinyl, 1,4oxazinyl, 2H-1,2-oxazinyl, 4H-1,4-oxazinyl, 1,2,5-oxathiazinyl, 1,2,6-oxathiazinyl, and the like.

Illustrative values for heterocyclic substituents that are fused benz derivatives of five- or six-membered heterocyclic radicals include anthranilyl, cinnolinyl, indolyl, isoindolyl, indenyl, isoindenyl, indolinyl, indolizinyl, benzofuranyl, 2H-1,3-benzoxazinyl, 2H-1,4-benzoxazinyl, 1H-2,3-benzoxazinyl, 4H-3,1-benzoxazinyl, 2H-1,2-benzoxazinyl, 4H-1,4-benzoxazinyl, benzoxazolyl, 2H-benzopyranyl, benzothienyl, benzimidazolyl, benzthiazolyl, carbazolyl, cyclopenta(b)pyridinyl, quinolinyl, isobenzofuryl, 1H-indazolyl, indoxazinyl, isoquinolinyl, quinazolinyl, quinoxalinyl, 1,8-naphthyridinyl, phthalazinyl, phenothiazinyl, acridinyl, phenoxazinyl, purinyl, pteridinyl, pyrano[3,4-b]pyrrolyl, pyrido[3,4-b]pyridinyl, pyrido[3,2-b]pyridinyl, pyrido[3,4-b]pyridinyl, and the like.

Illustrative values of (C₁-C₁₀)alkyl include methyl, ethyl, propyl, isopropyl, isobutyl, butyl, *tert*-butyl, pentyl, hexyl and the like.

Illustrative values for (C₁-C₃)perfluoroalkyl include trifluoromethyl, pentafluoroethyl and heptafluoropropyl.

Illustrative values for (C₁-C₃)perfluoroalkoxy include trifluoromethoxy, pentafluoroethoxy.

The compounds of formula (I), the stereoisomers and hydrates thereof, and the pharmaceutically acceptable salts of the compounds, stereoisomers and hydrates, may be prepared according to the preparative methodologies disclosed in the aforementioned commonly assigned PCT International Application Publication Nos. WO 96/40640 and WO 98/23593.

A preferred subgroup of formula (la) compounds are those compounds wherein R^{b} and R¹ are hydrogen, R² is 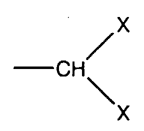 or -C(R^{a} R^{a})ₙ-X; and each X is independently aryl or heteroaryl.

A further preferred subgroup of formula (la) compounds are those wherein R² is -C(R^{a} R^{a})ₙ-X, each R^{a} being independently methyl, ethyl, or hydrogen; and X is phenyl or pyridyl.

An especially preferred subgroup of formula (la) compounds includes those compounds selected from the group consisting of:
7-[(4'-trifluoromethyl-biphenyl-2-carbonyl)-amino]-quinoline-3-carboxylic acid-(diphenylpyridin-2-yl-methyl)-amide;
7-[(4'-trifluoromethyl-biphenyl-2-carbonyl)-amino]-quinoline-3-carboxylic acid-(phenylpyridin-2-yl-methyl)-amide;
7-[(4'-trifluoromethyl-biphenyl-2-carbonyl)-amino]-quinoline-3-carboxylic acid(1-methyl-1-phenyl-ethyl)-amide;
7-[(4'-trifluoromethyl-biphenyl-2-carbonyl)-amino]-quinoline-3-carboxylic acid(1-phenyl-propyl)-amide;
(R)-7-[(4'-trifluoromethyl-biphenyl-2-carbonyl)-amino]-quinoline-3-carboxylic acid-(1-phenyl-ethyl)-amide;
7-[(4'-trifluoromethyl-biphenyl-2-carbonyl)-amino]-quinoline-3-carboxylic acid(1-pyridin-2-yl-propyl)-amide;
7-[(4'-trifluoromethyl-biphenyl-2-carbonyl)-amino]-quinoline-3-carboxylic acid-(2-pyridin-2-yl-ethyl)-amide;
7-[(4'-trifluoromethyl-biphenyl-2-carbonyl)-amino]-quinoline-3-carboxylic acid-ethylamide;
7-[(4'-trifluoromethyl-biphenyl-2-carbonyl)-amino]-quinoline-3-carboxylic acid-isopropylamide; and
7-[(4'-trifluoromethyl-biphenyl-2-carbonyl)-amino]-quinoline-3-carboxylic acid-diethylamide; the stereoisomers and prodrugs thereof, and the pharmaceutically acceptable salts of the compounds, stereoisomers, and prodrugs thereof.

In reference to both formula (I) and (la) compounds, the term "prodrug" refers to compounds that are drug precursors which, following administration, release the drug *in vivo* via a chemical or physiological process (e.g. a prodrug on being brought to physiological pH is converted to the desired drug form). Exemplary prodrugs, for example, release the corresponding free carboxylic acid, and such hydrolyzable ester-forming residues of the compounds of the invention include, but are not limited to, carboxylic acids wherein the free hydrogen atom is replaced by (C₁-C₆)alkyl, (C₂-C₁₂)alkanoylmethyl, (C₄-C₉)-1-(alkanoyloxy)ethyl, 1-methyl-1-(alkanoyloxy)-ethyl having from 5 to 10 carbon atoms, alkoxycarbonyloxymethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, γ-butyrolacton-4-yl, di-N,N-(C₁-C₂)alkylamino(C₂-C₃)alkyl (such as β-dimethylaminoethyl), carbamoyl-(C₁-C₂)alkyl, N,N-di(C₁-C₂)alkylcarbamoyl-(C₁-C2)alkyl and piperidino-, pyrrolidino-, or morpholino(C₁-C₂)alkyl.

Prodrugs of the invention where the carboxyl group in a carboxylic acid functionality has been derivatized as an ester may be prepared by combining the carboxylic acid with an appropriate alkyl halide in the presence of a base such as potassium carbonate in a reaction-inert solvent such as DMF at a temperature of about 0° C to about 100° C for about 1 to about 24 hours. Alternatively, the acid may be combined with an appropriate alcohol as the solvent in the presence of a catalytic amount of an acid such as concentrated sulfuric acid at a temperature of about 20° C to about 120° C, preferably at reflux, for about 1 hour to about 24 hours. Another method is to react the acid in an inert solvent such as THF, with concomitant removal of the water being produced by physical (e.g. a Dean-Stark trap), or chemical (e.g. molecular sieves) means.

Other exemplary prodrugs release an alcohol wherein the free hydrogen atom of the hydroxyl substituent of the metabolite is replaced by (C₁-C₆)alkanoyloxymethyl, 1-((C₁-C₆)alkanoyloxy)ethyl, 1-methyl-1-((C₁-C₆)alkanoyloxy)ethyl, (C₁-C₆)alkoxycarbonyloxymethyl, N-(C₁-C₆)alkoxycarbonylaminomethyl, succinoyl, (C₁-C₆)alkanoyl, a-amino(C₁-C₄)alkanoyl, arylacetyl, and α-aminoacyl, or α-aminoacyl-α-aminoacyl wherein the α-aminoacyl moieties are independently any of the naturally occurring L-amino acids found in proteins, -P(O)(OH)₂, -P(O)(O(C₁-C₆)alkyl)₂, or glycosyl (e.g. the radical resulting from detatchment of the hydroxyl of the hemiacetal of a carbohydrate).

Prodrugs of the invention where an alcohol functionality has been derivatized as an ether may be prepared by combining the alcohol with an appropriate alkyl bromide or iodide in the presence of a base such as potassium carbonate in a reaction-inert solvent such as DMF at a temperature of about 0° C to about 100° C for about 1 to about 24 hours. Alkanoylaminomethyl ethers may be obtained by reaction of the alochol with a bis-(alkanoylamino)methane in the presence of a catalytic amount of acid in a reaction-inert solvent such as THF, according to the method described in U.S. Pat. No. 4,997,984. Alternatively, these compounds may be prepared according to the methods described by Hoffman et al., in J. Organic Chem., 59, 3530 (1994).

The compounds of formula (la), the stereoisomers and prodrugs thereof, and the pharmaceutically acceptable salts of the compounds, stereoisomers, and prodrugs, may be prepared as illustrated in the reaction schemes outlined hereinbelow. These indicated processes may be effected in sequential, or convergent, synthetic routes. Purification procedures may comprise recrystallization and normal phase or reverse phase chromatography.

As a general note, the preparation of formula (la) compounds may require the protection of remote functionalities (e.g., primary amine, secondary amine, carboxyl). The need for such protection will vary depending on the nature of the remote functionality and the conditions of the preparative methods employed. The need for such protection is readily determined by one skilled in the art. The use of such protection/deprotection methods is also within the purview of one of ordinary skill in the art. For a general description of protecting groups and their use, see T.W. Greene and P.G.M. Wuts, Protective Groups in Organic Synthesis, John Wiley & Sons, New York, 1991.

The following abbreviations and/or acronyms are employed herein:
- ms: mass spectra
- APCI: atmospheric pressure chemical ionization
- NMR: nuclear magnetic resonance
- h: hour(s)
- d: day(s)
- min: minute(s)
- EDTA: ethylene diaminetetraacetic acid
- Triton-X®: polyoxyethyene ether
- SDS: sodium dodecyl sulfate
- SDS-PAGE: sodium dodecyl sulfate-polyacrylamide gel electrophoresis
- g: gram(s)
- mmol: millimoles
- mp: melting point
- NF: national formulary

### General Synthetic Procedure

The compounds of formula (la) may be prepared as illustrated in Scheme 1 hereinbelow. The quinoline II can be prepared by the method of C.C. Price, et al., Journal of the American Chemical Society, 69, 374-376 (1947). Conversion of quinoline II to chloroquinoline III can be achieved by treatment with oxalyl chloride. After recrystallization, chloroquinoline III can be reduced to aminoquinoline IV by reaction with ammonium formate and palladium on carbon. Reaction of amine IV with 4'-trifluoromethyl-biphenyl-2-carbonyl chloride affords amine V, which can be hydrolyzed to carboxylic acid VI by treatment with lithium hydroxide. Amides of formula (la) can be obtained by condensation of carboxylic acid VI with the appropriate amine VII under the usual amide coupling conditions known to those skilled in the art, such as reaction with N,N'-dialkylcarbodiimide (preferably 1-(3-dimethylaminopropyl)-3-ethylcarbodiimide hydrochloride [EDCI]), 1-hydroxybenzotriazole (HOBT), and an amine base (preferably triethylamine [TEA]) in a polar solvent (preferably dichloromethane) for a time between 1 and 100 hours (preferably overnight), at a temperature between 0° and 100°C (preferably ambient temperature). Amines of formula VII can be obtained from commercial sources, or prepared by a variety of methods known to those skilled in the art, including for example, those disclosed in various synthetic organic texts. Methods for obtaining amines of formula VII in enantiomerically enriched form, if not available from commercial sources, are known to those skilled in the art and include resolution by selective crystallization of a diastereomeric salt of an enantiomerically pure chiral acid (e.g., A. Ault, Organic Syntheses, Collective Volumes V: 932-936 (1973)) or an enantioselective synthesis such as those described by G. Alvara et al., Journal of the Chemical Society, Perkin Transactions 1: 777-783 (1998). Additionally, amines VII or amides I, if chiral, can be obtained in enantiomerically pure form by resolving the enatiomers of the racemate by preparative chiral HPLC (high-pressure liquid chromatography). Such methods will be well known, or apparent in light of the disclosure herein, to one of ordinary skill in the art.

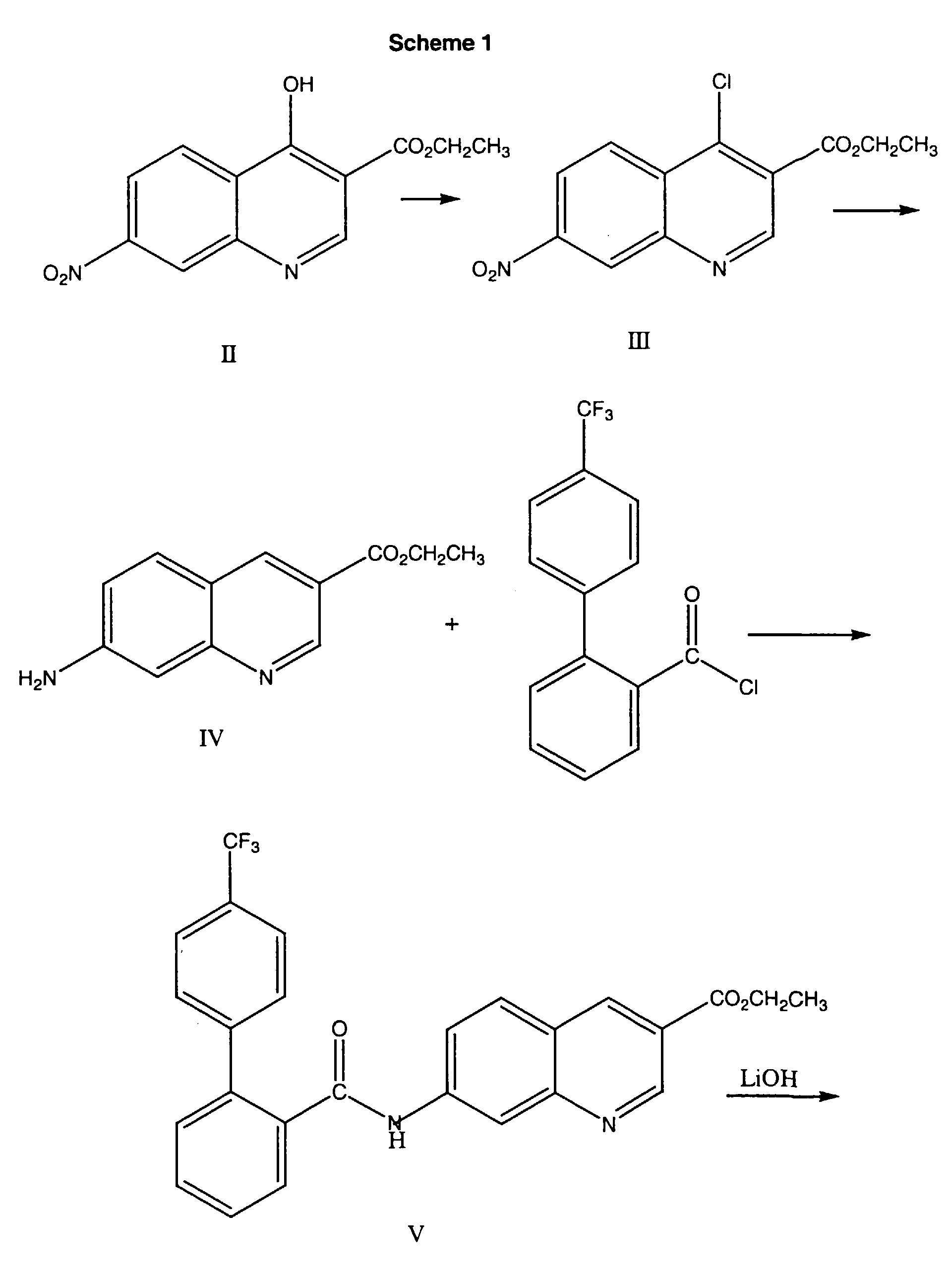

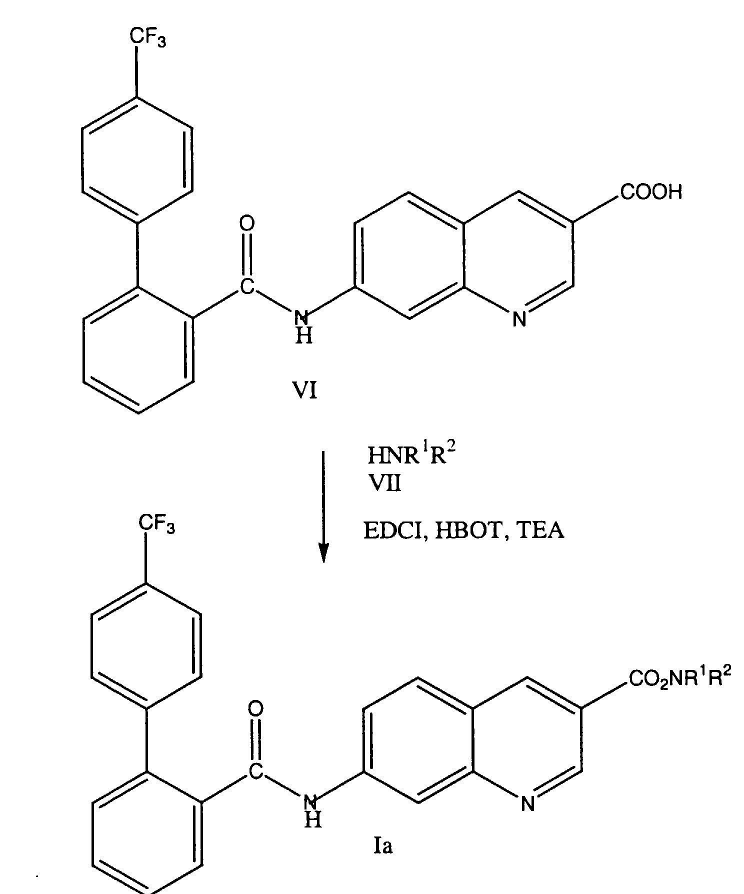

### Preparation 1

### 4-Chloro-7-nitro-quinoline-3-carboxylic acid ethyl ester (III)

4-Hydroxy-7-nitro-quinoline-3-carboxylic acid ethyl ester (15.6 g, 59.5 mmol) was suspended in chloroform (250 ml) as oxalyl chloride (20.7 ml, 30.2 g, 238 mmol) was added, followed by dimethylformamide (0.4 ml, 0.38 g, 5.2 mmol). After heating at reflux for 2.5 h, the mixture was added to 300 ml of a 2N aqueous sodium hydroxide solution cooled in an ice/water bath. After stirring vigorously for 30 minutes, the aqueous layer was extracted with chloroform (200 ml). The combined organic phases were washed with water, brine and then dried over anhydrous magnesium sulfate. After vacuum filtration, the solution was concentrated under vacuum to provide 15.6 g of a fluffy brown solid. This solid was transferred to a Soxhlet thimble and extracted with dichloromethane for 4 h employing a Soxhlet extractor. Concentration of the dichloromethane solution under vacuum yielded 14.68 g of a tan solid. This solid was crystallized by dissolving in 400 ml of hot acetone, cooling to 0°C overnight. The solid was collected by filtration, rinsing with ice-cold acetone, to yield 9.91 g of the title product as light yellow needles. MS (APCI) 281 and 283 (M+1 )⁺; ¹H NMR (CDCI₃) 1.45 (t, 3H, J = 7.0 Hz), 4.50 (q, 2H, J = 7.0 Hz), 8.42 (dd, 1H, J = 9.3, 2.2 Hz), 8.55 (d, 1H, J = 9.3 Hz), 8.98 (d, 1H, J = 2.2 Hz), 9.29 (s, 1H).

### Preparation 2

### 7-Amino-quinoline-3-carboxylic acid ethyl ester (IV)

4-Chloro-7-nitro-quinoline-3-carboxylic acid ethyl ester (14.57 g, 51.9 mmol) was suspended in methanol (210 ml) as palladium on carbon (10% palladium on carbon combined with an equal weight of water, 2.91 g) was added, followed by ammonium formate (13.09 g, 208 mmol). After heating at reflux for 3 h, the mixture was filtered through Celite® while still warm, rinsing with additional methanol to elute the color from the Celite® . Concentration of the filtrate under vacuum yielded 13.6 g of a yellow solid. This material was combined with 350 ml of acetone and the resulting slurry stirred for 1 h before filtration and concentration of the filtrate yielded 10.85 g of the title compound as a yellow solid. MS (APCI) 217 (M+1)⁺ ; ¹H NMR (DMSO-d₆) 1.34 (t, 3H, J = 7.2 Hz), 4.34 (q, 2H, J = 7.2 Hz), 6.34 (bs, 2H), 6.96 (d, 1H, J = 2.0 Hz), 7.06 (dd, 1H, J = 8.9, 2.3 Hz), 7.81 (d, 1H, J = 8.9 Hz), 8.59 (d, 1H, J = 2.0 Hz), 9.03 (d, 1H, J = 2.0 Hz).

### Preparation 3

### 4'-Trifluoromethyl-biphenyl-2-carbonyl chloride

4'-Trifluoromethyl-biphenyl-2-carboxylic acid (25 g, 94 mmol) was combined with thionyl chloride (35 ml, 470 mmol) and the mixture was heated at reflux. After 2 h, the mixture was concentrated under vacuum to afford 26.5 g of the title product as a light yellow oil. ¹H NMR (CDCI₃) 7.37 (dd, 1H, J = 7.6, 1.1Hz), 7.43 (d, 2H, J = 8.1 Hz), 7.55 (td, 1H, J = 7.7, 1.3 Hz), 7.66 (td, 1H, J = 7.5, 1.3 Hz), 7.68 (d, 2H, J = 8.1 Hz), 8.11 (dd, 1H, J = 7.9, 1.2 Hz).

### Preparation 4

### 7-[(4'-Trifluoromethyl-biphenyl-2-carbonyl)-amino]-quinoline-3-carboxylic acid ethyl ester (V)

7-Amino-quinoline-3-carboxylic acid ethyl ester (8.6 g, 39.8 mmol) was combined with pyridine (12.9 ml, 159 mmol) and 4-N,N-dimethylamino-pyridine (0.5 g, 4 mmol) in 100 ml of chloroform. The mixture was stirred as 4'-trifluoromethyl-biphenyl-2-carbonyl chloride (22.64 g, 79.5 mmol) was added as a solution in 100 ml of chloroform. After heating at reflux for 2 h, the mixture was concentrated under vacuum, the residue taken up in ethyl acetate (600 ml) and washed sequentially with a 1 N aqueous hydrochloric acid solution (2 x 200 ml), water, and brine. The organic phase was then dried over anhydrous magnesium sulfate, filtered and concentrated under vacuum to yield 29.4 g of a red oil. This oil was purified by chromatography on silica, eluting sequentially with a 70:30 dichloromethane/hexanes solution, followed by dichloromethane, then a 10:90 ethyl acetate/dichloromethane solution. Concentration under vacuum of the product-containing fractions afforded 13.15 g of the title compound as a yellow solid. MS (APCI) 465 (M+1)⁺; 463 (M-1)⁻; ¹H NMR (DMSO-d₆) 1.35 (t, 3H, J = 7.0 Hz), 4.37 (q, 2H, J = 7.0 Hz), 7.50-7.73 (m, 9H), 8.09 (d, 1H, J = 9.0 Hz), 8.43 (s, 1H), 8.85 (s, 1H), 9.22 (d, 1H, J = 1.9 Hz), 10.9 (s, 1H).

### Preparation 5

### 7-[(4'-Trifluoromethyl-biphenyl-2-carbonyl)-amino]-quinoline-3-carboxylic acid (VI)

7-[(4'-Trifluoromethyl-biphenyl-2-carbonyl)-amino]-quinoline-3-carboxylic acid ethyl ester (10.28 g, 22.1 mmol) and lithium hydroxide monohydrate (1.86 g, 44.3 mmol) were added to 125 ml of a solution consisting of a 3:1:1 volume ratio of tetrahydrofuran, methanol, and water. After stirring for 3.5 h at ambient temperature, the mixture was concentrated under vacuum to yield an aqueous emulsion of a yellow oil. More water (100 ml) was added and with efficient stirring, the aqueous mixture was made acidic (pH 2) with a 1 N aqueous hydrochloric acid solution. Collection of the resulting solid by vacuum filtration, maintaining the flow of air overnight to dry the solid, and drying under vacuum afforded 9.0 g of the title compound as a yellow powder. MS (APCI) 437 (M+1)⁺; 435 (M-1)⁻; ¹H NMR (DMSO-d₆) 7.50-7.72 (m, 9H), 8.05 (d, 1H, J = 9.0 Hz), 8.40 (s, 1H), 8.80 (d, 1H, J = 2.1 Hz), 10.85 (s, 1H).

The compounds of formula (la) were prepared as outlined in the following examples beginning with the intermediate 7-[(4'-trifluoromethyl-biphenyl-2-carbonyl)-amino]-quinoline-3-carboxylic acid (VI).

### Example 1

### 7-[(4'-Trifluoromethyl-biphenyl-2-carbonyl)-amino]-quinoline-3-carboxylic acid (dipyridin-2-yl-methyl)-amide

7-[(4'-Trifluoromethyl-biphenyl-2-carbonyl)-amino]-quinoline-3-carboxylic acid (250 mg, 0.57 mmol) was combined with di-(2-pyridyl)-methylamine hydrochloride (126 mg, 0.57 mmol), 1-(3-dimethylaminopropyl)-3-ethylcarbodiimide hydrochloride (121 mg, 0.69 mmol), 1-hydroxybenzotriazole (85 mg, 0.63 mmol), and triethylamine (0.32 ml, 2.3 mmol) in 3 ml of dichloromethane. After stirring overnight at ambient temperature, the reaction mixture was diluted with 75 ml of dichloromethane, and the organic phase washed sequentially with water (2 x 40 ml), and brine, and then dried over anhydrous magnesium sulfate, filtered and concentrated under vacuum to provide a yellow residue. This material was purified by preparative thin-layer chromatography on silica eluting with 3% methanol in dichloromethane. The product-containing band was collected and eluted with 5% methanol in ethyl acetate to afford 112 mg of the title compound as a colorless solid. MS (APCI) 604 (M+1)⁺; 602 (M-1)⁻; ¹H NMR (DMSO-d₆) 6.48 (d, 1H, J = 7.9 Hz), 7.26 (ddd, 2H, J = 7.5, 4.8, 1.0 Hz), 7.53-7.72 (m, 11H), 7.76 (td, 2H, J = 7.7, 1.7 Hz), 7.98 (d, 1H, J = 8.9 Hz), 8.39 (d, 1H, J = 1.5 Hz), 8.49 (ddd, 2H, J = 4.8, 1.9, 0.9 Hz), 8.84 (d, 1H, J = 2.0 Hz), 9.22 (d, 1H, J = 2.0 Hz), 9.53 (d, 1H, J = 7.9 Hz), 10.8 (s, 1H).

### Example 1 A

### 7-[(4'-Trifluoromethyl-biphenyl-2-carbonyl)-amino]-quinoline-3-carboxylic acid (dipyridin-2-yl-methyl)-amide, ethanesulfonate

Trifluoromethyl-biphenyl-2-carbonyl)-amino]-quinoline-3-carboxylic acid (dipyridin-2-yl-methyl)-amide (112 mg, 0.185 mmol) was dissolved in 5 ml of ethyl acetate, and ethanesulfonic acid (20 mg, 1 equivalent) was added as a solution in 1 ml of diethyl ether. After 30 min, the mixture was concentrated under vacuum to afford 120 mg of the title compound as a light yellow solid. MS (APCI) 604 (M+1)⁺; 602 (M-1)⁻; ¹H NMR (DMSO-d₆) 1.05 (t, 3H, J = 7.3 Hz), 2.38 (q, 2H, J = 7.3 Hz), 6.67 (d, 1H, J = 7.3 Hz), 7.48-7.79 (m, 13H), 8.02 (td, 2H, J = 7.8, 1.7 Hz), 8.13 (d, 1H, J = 8.9 Hz), 8.58 (s, 1H), 8.64 (dd, 2H, J = 5.0, 1.0 Hz), 9.09 (s, 1H), 9.37 (d, 1H, J = 2.3 Hz), 9.84 (d, 1H, J = 7.6 Hz), 11.05 (s, 1H).

### Example 1 B

### 7-[(4'-Trifluoromethyl-biphenyl-2-carbonyl)-amino]-quinoline-3-carboxylic acid (dipyridin-2-yl-methyl)-amide, bis-ethanesulfonate

7-[(4'-Trifluoromethyl-biphenyl-2-carbonyl)-amino]-quinoline-3-carboxylic acid (dipyridin-2-yl-methyl)-amide (140 mg, 0.232 mmol) was dissolved in 10 ml of ethyl acetate, and ethanesulfonic acid (25 mg, 2 equivalents) was added as a solution in 1.5 ml of diethyl ether. After 15 min, 5 ml more of diethyl ether was added. After stirring for another hour, the mixture was vacuum filtered to afford 85 mg of the title compound as a light yellow solid. MS (APCI) 604 (M+1)⁺; 602 (M-1)⁻ ; ¹H NMR (DMSO-d₆) 1.05 (t, 6H, J = 7.4 Hz), 2.40 (q, 4H, J = 7.4 Hz), 6.72 (d, 1H, J = 7.2 Hz), 7.55-7.84 (m, 13H), 8.11 (td, 2H, J = 7.9, 1.7 Hz), 8.19 (d, 1H, J = 8.9 Hz), 8.65-8.69 (m, 3H), 9.20 (s, 1H), 9.42 (d, 1H, J = 2.0 Hz), 9.97 (d, 1H, J = 7.2 Hz), 11.15 (s, 1H).

### Example 2

### 7-[(4'-Trifluoromethyl-biphenyl-2-carbonyl)-amino]-quinoline-3-carboxylic acid (phenyl-pyridin-2-yl-methyl)-amide

7-[(4'-Trifluoromethyl-biphenyl-2-carbonyl)-amino]-quinoline-3-carboxylic acid (100 mg, 0.23 mmol) was combined with phenyl-(2-pyridyl)-methylamine (42 mg, 0.23 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was diluted with 50 ml of dichloromethane, and the organic phase washed sequentially with water (2 x 20 ml), and brine, and then dried over anhydrous magnesium sulfate, filtered and concentrated under vacuum to provide a yellow residue. This material was purified by preparative thin-layer chromatography on silica eluting with ethyl acetate. The product-containing band was collected and eluted with 5% methanol in ethyl acetate to afford 63 mg of the title compound as a colorless solid. MS (APCI) 603 (M+1)⁺; 601 (M-1)⁻; ¹H NMR (DMSO-d₆) 6.48 (d, 1H, J = 7.9 Hz), 7.25-7.38 (m, 4H), 7.44 (d, 2H, J = 7.2 Hz) 7.55-7.85 (m, 11H), 8.02 (d, 1H, J = 9.2 Hz), 8.43 (s, 1H), 8.56 (d, 1H, J = 4.6 Hz), 8.85 (d, 1H, J = 1.7 Hz), 9.25 (d, 1H, J = 2.3 Hz), 9.56 (d, 1H, J = 8.2 Hz), 10.85 (s, 1H).

### Example 2A

### 7-[(4'-Trifluoromethyl-biphenyl-2-carbonyl)-amino]-quinoline-3-carboxylic acid (phenyl-pyridin-2-yl-methyl)-amide, ethanesulfonate

7-[(4'-Trifluoromethyl-biphenyl-2-carbonyl)-amino]-quinoline-3-carboxylic acid (phenyl-pyridin-2-yl-methyl)-amide (63 mg, 0.105 mmol) was dissolved in 5 ml of ethyl acetate, and ethanesulfonic acid (12 mg, 1 equivalent) was added as a solution in 1 ml of diethyl ether. After 30 min, the mixture was concentrated under vacuum to afford 70 mg of the title compound as a light yellow solid. MS (APCI) 603 (M+1)⁺; 601 (M-1)⁻; ¹H NMR (DMSO-d₆) 1.01 (t, 3H, J = 7.3 Hz), 2.37 (q, 2H, J = 7.3 Hz), 6.53 (d, 1H, J = 7.3 Hz), 7.28-7.79 (m, 16H), 8.07 (t, 1H, J = 7.7 Hz), 8.18 (d, 1H, J = 9.0 Hz), 8.65-8.66 (m, 2H), 9.20 (s, 1H), 9.40 (s, 1H), 9.80 (d, 1H), 11.15 (s, 1H).

### Example 2B

### (S)-7-[(4'-Trifluoromethyl-biphenyl-2-carbonyl)-amino]-quinoline-3-carboxylic acid (phenyl-pyridin-2-yl-methyl)-amide

7-[(4'-Trifluoromethyl-biphenyl-2-carbonyl)-amino]-quinoline-3-carboxylic acid (100 mg, 0.23 mmol) was combined with (S)-phenyl-(2-pyridyl)-methylamine (64 mg, 0.35 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was diluted with 50 ml of dichloromethane, and the organic phase washed sequentially with water (2 x 20 ml), and brine, and then dried over anhydrous magnesium sulfate, filtered and concentrated under vacuum to provide a yellow residue. This material was purified by preparative thin-layer chromatography on silica eluting with ethyl acetate. The product-containing band was collected and eluted with 5% methanol in ethyl acetate to afford 86 mg of the title compound as a colorless solid. Optical Rotation: [α]_{D} = +44.1° (c = 0.39 mg/ml; CH₃OH); MS (APCI) 603 (M+1)⁺; 601 (M-1)⁻; ¹H NMR (DMSO-d₆) 6.48 (d, 1H, J = 7.9 Hz), 7.25-7.38 (m, 4H), 7.44 (d, 2H, J = 7.2 Hz) 7.55-7.85 (m, 11H), 8.02 (d, 1H, J = 9.2 Hz), 8.43 (s, 1H), 8.56 (d, 1H, J = 4.6 Hz), 8.85 (d, 1H, J = 1.7 Hz), 9.25 (d, 1H, J = 2.3 Hz), 9.56 (d, 1H, J = 8.2 Hz),10.85 (s, 1H).

### Example 2C

### (S)-7-[(4'-Trifluoromethyl-biphenyl-2-carbonyl)-amino]-quinoline-3-carboxylic acid (phenyl-pyridin-2-yl-methyl)-amide, ethanesulfonate:

(S)-7-[(4'-Trifluoromethyl-biphenyl-2-carbonyl)-amino]-quinoline-3-carboxylic acid (phenyl-pyridin-2-yl-methyl)-amide (63 mg, 0.105 mmol) was dissolved in 5 ml of ethyl acetate, and ethanesulfonic acid (12 mg, 1 equivalent) was added as a solution in 1 ml of diethyl ether. After 30 min, the mixture was concentrated under vacuum to afford 70 mg of the title compound as a light yellow solid. MS (APCI) 603 (M+1)⁺; 601 (M-1)⁻; ¹H NMR (DMSO-d₆) 6.48 (d, 1H, J = 7.9 Hz), 7.25-7.38 (m, 4H), 7.44 (d, 2H, J = 7.2 Hz) 7.55-7.85 (m, 11H), 8.02 (d, 1H, J = 9.2 Hz), 8.43 (s, 1H), 8.56 (d, 1H, J = 4.6 Hz), 8.85 (d, 1H, J = 1.7 Hz), 9.25 (d, 1H, J = 2.3 Hz), 9.56 (d, 1H, J = 8.2 Hz), 10.85 (s, 1H).

### Example 2D

### (S)-7-[(4'-Trifluoromethyl-biphenyl-2-carbonylfamino]-quinoline-3-carboxylic acid (phenyl-pyridin-2-yl-methyl)-amide, bis-ethanesulfonate

**(*S*)-7-[(4'-Trifluoromethyl-biphenyl-2-carbonyl)-amino]-quinoline-3-carboxylic** acid (phenyl-pyridin-2-yl-methyl)-amide (250 mg, 0.415 mmol) was dissolved in 15 ml of ethyl acetate, and ethanesulfonic acid (114 mg, 2.5 equivalents) was added as a solution in 5 ml of ethyl acetate. After 1 h, the mixture was concentrated under vacuum, suspended in 30 ml of diethyl ether, and the slurry vacuum filtered to afford 351 mg of the title compound as a light yellow solid. This material was dissolved in 3 ml of ethanol and then 60 ml of ethyl acetate was added gradually. After stirring overnight, the resulting solid was collected by vacuum filtration to afford 289 mg of the title compound as a colorless solid (mp = 158 °C). MS (APCI) 603 (M+1)⁺; 601 (M-1)⁻; ¹H NMR (DMSO-d₆) 1.06 (t, 6H, J = 7.3 Hz), 2.43 (q, 4H, J = 7.3 Hz), 6.57 (d, 1H, J = 7.3 Hz), 7.32-7.83 (m, 16H), 8.16 (t, 1H, J = 7.4 Hz), 8.24 (d, 1H, J = 9.2 Hz), 8.72-8.74 (m, 2H), 9.29 (s, 1H), 9.46 (d, 1H, J = 2.0 Hz), 9.89 (d, 1H, J = 7.2 Hz), 11.2 (s, 1H).

### Example 2E

### (R)-7-[(4'-Trifluoromethyl-biphenyl-2-carbonyl)-amino]-quinoline-3-carboxylic acid (phenyl-pyridin-2-yl-methyl)-amide

7-[(4'-Trifluoromethyl-biphenyl-2-carbonyl)-amino]-quinoline-3-carboxylic acid (100 mg, 0.23 mmol) was combined with (R)-phenyl-(2-pyridyl)-methylamine (64 mg, 0.35 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was diluted with 50 ml of dichloromethane, and the organic phase washed sequentially with water (2 x 20 ml), and brine, and then dried over anhydrous magnesium sulfate, filtered and concentrated under vacuum to provide a yellow residue. This material was purified by preparative thin-layer-chromatography on silica eluting with ethyl acetate. The product-containing band was collected and eluted with 5% methanol in ethyl acetate to afford 73 mg of the title compound as a colorless solid. Optical Rotation: [α]_{D} = -45.0° (c = 0.40 mg/ml; CH₃OH); MS (APCI) 603 (M+1)⁺; 601 (M-1)⁻ ; ¹H NMR (DMSO-d₆) 6.48 (d, 1H, J = 7.9 Hz), 7.25-7.38 (m, 4H), 7.44 (d, 2H, J = 7.2 Hz) 7.55-7.85 (m, 11H), 8.02 (d, 1H, J = 9.2 Hz), 8.43 (s, 1H), 8.56 (d, 1H, J = 4.6 Hz), 8.85 (d, 1H, J = 1.7 Hz), 9.25 (d, 1H, J = 2.3 Hz), 9.56 (d, 1H, J = 8.2 Hz), 10.85 (s, 1H).

### Example 2F

### (R)-7-[(4'-Trifluoromethyl-biphenyl-2-carbonyl)-amino]-quinoline-3-carboxylic acid (phenyl-pyridin-2-yl-methyl)-amide, ethanesulfonate

(*R*)-7-[(4'-Trifluoromethyl-biphenyl-2-carbonyl)-amino]-quinoline-3-carboxylic acid (phenyl-pyridin-2-yl-methyl)-amide (63 mg, 0.105 mmol) was dissolved in 5 ml of ethyl acetate, and ethanesulfonic acid (11 mg, 1 equivalent) was added as a solution in 1 ml of diethyl ether. After 30 min, the mixture was concentrated under vacuum to afford 65 mg of the title compound as a light yellow solid. MS (APCI) 603 (M+1)⁺; 601 (M-1)⁻; ¹H NMR (DMSO-d₆) 6.48 (d, 1H, J = 7.9 Hz), 7.25-7.38 (m, 4H), 7.44 (d, 2H, J = 7.2 Hz) 7.55-7.85 (m, 11H), 8.02 (d, 1H, J = 9.2 Hz), 8.43 (s, 1H), 8.56 (d, 1H, J = 4.6 Hz), 8.85 (d, 1H, J = 1.7 Hz), 9.25 (d, 1H, J = 2.3 Hz), 9.56 (d, 1H, J = 8.2 Hz), 10.85 (s, 1H).

### Example 3

### 7-[(4'-Trifluoromethyl-biphenyl-2-carbonyl)-amino]-quinoline-3-carboxylic acid (1-carbamoyl-2-phenyl-ethyl)-amide

7-[(4'-Trifluoromethyl-biphenyl-2-carbonyl)-amino]-quinoline-3-carboxylic acid (100 mg, 0.23 mmol) was combined with phenylalaninamide (38 mg, 0.23 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was diluted with 50 ml of dichloromethane, and the organic phase washed sequentially with water, and brine, and then dried over anhydrous magnesium sulfate, filtered and concentrated under vacuum to provide a residue. This material was purified by preparative thin-layer chromatography on silica eluting with ethyl acetate. The product-containing band was collected and eluted with 5% methanol in ethyl acetate to afford 47 mg of the title compound. MS (APCI) 583 (M+1)⁺; 581 (M-1)⁻ ; ¹H NMR (DMSO-d₆) 2.96 (dd, 1H, J = 13.1, 11.0 Hz), 3.14 (dd, 1H, J = 13.7, 3.7 Hz), 4.64-4.70 (m, 1H), 7.11-7.14 (m, 2H), 7.22 (t, 2H, J = 7.7 Hz), 7.33 (d, 2H, J = 7.7 Hz), 7.52-7.73 (m, 10H), 7.95 (d, 1H, J = 8.9 Hz), 8.37 (s, 1H), 8.64 (s, 1H), 8.82 (d, 1H, J = 8.2 Hz), 9.10 (s, 1H), 10.85 (s, 1H).

### Example 4

### 7-[(4'-Trifluoromethyl-biphenyl-2-carbonyl)-amino]-quinoline-3-carboxylic acid (carbamoyl-phenyl-methyl)-amide

7-[(4'-Trifluoromethyl-biphenyl-2-carbonyl)-amino]-quinoline-3-carboxylic acid (100 mg, 0.23 mmol) was combined with phenylglycinamide (34 mg, 0.23 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was diluted with 50 ml of dichloromethane, and the organic phase washed sequentially with water, and brine, and then dried over anhydrous magnesium sulfate, filtered and concentrated under vacuum to provide a residue. This material was purified by preparative thin-layer chromatography on silica eluting with ethyl acetate. The product-containing band was collected and eluted with 5% methanol in ethyl acetate to afford 56 mg of the title compound. MS (APCI) 569 (M+1)⁺; 567 (M-1)⁻; ¹H NMR (DMSO-d₆) 5.62 (d, 1H, J = 7.9 Hz), 7.21-7.34 (m, 4H), 7.50-7.71 (m, 12H), 7.95 (d, 1H, J = 9.1 Hz), 8.36 (s, 1H), 8.79 (d, 1H, J = 1.6 Hz), 9.07 (d, 1H, J = 8.1 Hz), 9.17 (d, 1H, J = 2.3 Hz), 10.85 (s, 1H).

### Example 5

### 7-[(4'-Trifluoromethyl_biphenyl-2-carbonyl)-amino]-quinoline-3-carboxylic acid propylamide

7-[(4'-Trifluoromethyl-biphenyl-2-carbonyl)-amino]-quinoline-3-carboxylic acid (100 mg, 0.23 mmol) was combined with propylamine (0.019 ml, 0.23 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was diluted with 50 ml of dichloromethane, and the organic phase washed sequentially with water, and brine, and then dried over anhydrous magnesium sulfate, filtered and concentrated under vacuum to provide a residue. This material was purified by preparative thin-layer chromatography on silica eluting with a 4:1 ethyl acetate-hexanes mixture. The product-containing band was collected and eluted with 5% methanol in ethyl acetate to afford 47 mg of the title compound. MS (APCI) 478 (M+1)⁺; 476 (M-1)⁻; ¹H NMR (DMSO-d₆) 0.87 (t, 3H, J = 7.4 Hz), 1.52 (hextet, 2H, J = 7.3 Hz), 3.23 (q, 2H, J = 7.0 Hz), 7.50-7.71 (m, 9H), 7.94 (d, 1H, J = 8.7 Hz), 8.37 (s, 1H), 8.64 (d, 1H, J = 1.9 Hz), 8.69 (t, 1H, J = 5.5 Hz), 9.15 (d, 1H, J =2.1 Hz), 10.85 (S, 1H).

### Example 6

### 7-[(4'-Trifluoromethyl-biphenyl-2-carbonyl)-amino]-quinoline-3-carboxylic acid (2,2,2-trifluoro-ethyl)-amide

7-[(4'-Trifluoromethyl-biphenyl-2-carbonyl)-amino]-quinoline-3-carboxylic acid (100 mg, 0.23 mmol) was combined with 2,2,2-trifluoroethylamine hydrochloride (31 mg, 0.23 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was concentrated, the residue was suspended in water, and the solid was collected by vacuum filtration to afford 72 mg of the title compound. MS (APCI) 518 (M+1)⁺; 516 (M-1)⁻; ¹H NMR (DMSO-d₆) 4.17 (dq, 2H, J = 9.8, 6.3 Hz), 7.56-7.76 (m, 9H), 8.03 (d, 1H, J = 8.9 Hz), 8.44 (d, 1H, J = 1.7 Hz), 9.23 (d, 1H, J = 2.0 Hz), 9.40 (t, 1H, J = 6.3 Hz), 10.85 (s, 1H).

### Example 7

### 7-[(4'-Trifluoromethyl-biphenyl-2-carbonyl)-amino]-quinoline-3-carboxylic acid (1-methyl-1-phenyl-ethyl)-amide

7-[(4'-Trifluoromethyl-biphenyl-2-carbonyl)-amino]-quinoline-3-carboxylic acid (100 mg, 0.23 mmol) was combined with 2-amino-2-phenylpropane (31 mg, 0.23 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was diluted with 50 ml of dichloromethane, and the organic phase washed sequentially with water, and brine, and then dried over anhydrous magnesium sulfate, filtered and concentrated under vacuum to provide a residue. This material was purified by preparative thin-layer chromatography on silica eluting with a 3:1 ethyl acetate-hexanes mixture. The product-containing band was collected and eluted with 5% methanol in ethyl acetate to afford 67 mg of the title compound as a colorless solid. MS (APCI) 554 (M+1)⁺; 552 (M-1)⁻; ¹H NMR (DMSO-d₆) 1.66 (s, 1H), 7.13 (m, 1H), 7.25 (m, 2H), 7.37 (d, 2H, J = 7.7 Hz), 7.50-7.70 (m, 9H), 7.95 (d, 1H, J = 8.7 Hz), 8.36 (s, 1H), 8.69 (s, 1H), 8.72 (s, 1H), 9.10 (s, 1H), 10.85 (s, 1H).

### Example 8

### 7-[(4'-Trifluoromethyl-biphenyl-2-carbonyl)-amino]-quinoline-3-carboxylic acid cyclopentylamide

7-[(4'-Trifluoromethyl-biphenyl-2-carbonyl)-amino]-quinoline-3-carboxylic acid (100 mg, 0.23 mmol) was combined with cyclopentylamine (20 mg, 0.23 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was diluted with 50 ml of dichloromethane, and the organic phase washed sequentially with water, and brine, and then dried over anhydrous magnesium sulfate, filtered and concentrated under vacuum to provide a residue. This material was purified by preparative thin-layer chromatography on silica eluting with a 3:1 ethyl acetate-hexanes mixture. The product-containing band was collected and eluted with 5% methanol in ethyl acetate to afford 54 mg of the title compound as a colorless solid. MS (APCI) 504 (M+1)⁺; 502 (M-1)⁻; ¹H NMR (DMSO-d₆) 1.50-1.59 (m, 4H), 1.60-1.75 (m, 2H), 1.80-1.95 (m, 2H), 4.20-4.30 (m, 1H), 7.50-7.71 (m, 9H), 7.94 (d, 1H, J = 8.9 Hz), 8.36 (s, 1H), 8.54 (d, 1H, J = 7.5 Hz), 8.64 (d, 1H, J = 2.1 Hz), 9.14 (d, 1H, J = 2.3 Hz), 10.85 (s, 1H).

### Example 9

### 7-[(4'-Trifluoromethyl-biphenyl-2-carbonyl)-amino]-quinoline-3-carboxylic acid (1-phenyl-propyl)-amide

7-[(4'-Trifluoromethyl-biphenyl-2-carbonyl)-amino]-quinoline-3-carboxylic acid (100 mg, 0.23 mmol) was combined with 1-phenylpropylamine (31 mg, 0.23 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was concentrated under vacuum, the residue was suspended in water, and the solid was collected by vacuum filtration. This material was purified by preparative thin-layer chromatography on silica eluting with a 3:1 ethyl acetate-hexanes mixture. The product-containing band was collected and eluted with 5% methanol in ethyl acetate to afford 66 mg of the title compound as a colorless solid. MS (APCI) 554 (M+1)⁺; 552 (M-1)⁻; ¹H NMR (DMSO-d₆) 0.89 (t, 3H, J = 7.3 Hz), 1.75-1.85 (m, 2H), 4.92 (td, 1H, J = 8.7, 6.2 Hz), 7.19 (t, 1H, J = 7.3 Hz), 7.29 (t, 2H, J = 7.6 Hz), 7.38 (d, 2H, J = 7.3 Hz), 7.50-7.75 (m, 8H), 7.97 (d, 1H, J = 8.9 Hz), 8.38 (s, 1H), 8.70 (d, 1H, J = 1.9 Hz), 9.00 (d, 1H, J = 8.3 Hz), 9.17 (d, 1H, J = 2.3 Hz), 10.85 (s, 1H).

### Example 10

### (R)-7-[(4'-Trifluoromethyl-biphenyl-2-carbonyl)-amino]-quinoline-3-carboxylic acid (1-phenyl-ethyl)-amide, ethanesulfonate

7-[(4'-Trifluoromethyl-biphenyl-2-carbonyl)-amino]-quinoline-3-carboxylic acid (100 mg, 0.23 mmol) was combined with (R)-1-phenylethylamine (28 mg, 0.23 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was concentrated, the residue was suspended in water, and the solid was collected by vacuum filtration. This material was purified by preparative thin-layer chromatography on silica eluting with a 3:1 ethyl acetate-hexanes mixture The product-containing band was collected and eluted with 5% methanol in ethyl acetate to afford 62 mg of product. This material was dissolved in 5 ml of ethyl acetate, and ethanesulfonic acid (12.5 mg, 1 equivalent) was added as a solution in 1 ml of diethyl ether. After 30 min, the mixture was concentrated under vacuum to afford 68 mg of the title compound as a yellow solid. MS (APCI) 540 (M+1)⁺; 538 (M-1)⁻; ¹H NMR (DMSO-d₆) 1.06 (t, 3H, J = 7.4 Hz), 1.53 (d, 3H, J = 6.9), 2.40 (q, 2H, J = 7.4 Hz), 5.24 (quintet, 1H, J = 7.1 Hz), 7.22-7.27 (m, 1H), 7.33-7.38 (m, 2H), 7.45 (d, 2H, J = 7.3 Hz), 7.56-7.81 (m, 9H), 8.18 (d, 1H, J = 8.9 Hz), 8.65 (s, 1H), 9.11 (s, 1H), 9.25 (d, 1H, J = 7.9 Hz), 9.38 (d, 1H, J = 2.0 Hz), 11.15 (s, 1H).

### Example 11

### 7-[(4'-Trifluoromethyl-biphenyl-2-carbonyl)-amino]-quinoline-3-carboxylic acid (1-pyridin-2-yl-propyl)-amide

7-[(4'-Trifluoromethyl-biphenyl-2-carbonyl)-amino]-quinoline-3-carboxylic acid (100 mg, 0.23 mmol) was combined with 1-(2-pyridyl)-propylamine (31mg, 0.23 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was diluted with 50 ml of dichloromethane, and the organic phase washed sequentially with water, and brine, and then dried over anhydrous magnesium sulfate, filtered and concentrated under vacuum to provide a residue. This material was purified by preparative thin-layer chromatography on silica eluting with 4% methanol in dichloromethane. The product-containing band was collected and eluted with 5% methanol in ethyl acetate to afford 65 mg of the title compound. MS (APCI) 555 (M+1)⁺; 553 (M-1)⁻; ¹H NMR (DMSO-d₆) 0.91 (t, 3H, J = 7.4 Hz), 1.84-1.94 (m, 2H), 5.00-5.03 (m, 1H), 7.22 (ddd, 1H, J = 7.5, 4.8, 1.0 Hz), 7.42 (d, 1H, J = 7.9 Hz), 7.51-7.74 (m, 1 OH), 7.97 (d, 1H, J = 8.9 Hz), 8.38 (d, 1H, J = 1.7 Hz), 8.49 (ddd, 1H, J = 4.8, 1.9, 1.0 Hz), 8.75 (d, 1H, J = 1.7 Hz), 9.02 (d, 1H, J = 8.1 Hz), 9.20 (d, 1H, J = 2.3 Hz), 10.82 (s, 1H).

### Example 11 A

### (R)-7-[(4'-Trifluoromethyl-biphenyl-2-carbonyl)-amino]-quinoline-3-carboxylic acid (1-pyridin-2-yl-propyl)-amide

7-[(4'-Trifluoromethyl-biphenyl-2-carbonyl)-amino]-quinoline-3-carboxylic acid (100 mg, 0.23 mmol) was combined with 1-(2-pyridyl)-propylamine hydrochloride (40 mg, 0.23 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was diluted with 50 ml of dichloromethane, and the organic phase washed sequentially with water, and brine, and then dried over anhydrous magnesium sulfate, filtered and concentrated under vacuum to afford 112 mg of the title compound as a light yellow solid. Optical Rotation: [α]_{D} = -66.8° (c = 0.40 mg/ml; CH₃OH). MS (APCI) 555 (M+1)⁺; 553 (M-1)⁻; ¹H NMR (DMSO-d₆) 0.91 (t, 3H, J = 7.4 Hz), 1.84-1.94 (m, 2H), 5.00-5.03 (m, 1H), 7.22 (ddd, 1H, J = 7.5, 4.8, 1.0 Hz), 7.42 (d, 1H, J = 7.9 Hz), 7.51-7.74 (m, 10H), 7.97 (d, 1H, J = 8.9 Hz), 8.38 (d, 1H, J = 1.7 Hz), 8.49 (ddd, 1H, J = 4.8, 1.9, 1.0 Hz), 8.75 (d, 1H, J = 1.7 Hz), 9.02 (d, 1H, J = 8.1 Hz), 9.20 (d, 1H, J = 2.3 Hz),10.82 (s, 1H).

### Example 11 B

### (R)-7-[(4'-Trifluoromethyl-biphenyl-2-carbonyl)-amino]-quinoline-3-carboxylic acid (1-pyridin-2-yl-propyl)-amide, ethanesulfonate

(R)-7-[(4'-Trifluoromethyl-biphenyl-2-carbonyl)-amino]-quinoline-3-carboxylic acid (1-pyridin-2-yl-propyl)-amide (99 mg) was dissolved in 5 ml of ethyl acetate, and ethanesulfonic acid (20 mg, 1 equivalent) was added as a solution in 1.5 ml of ethyl acetate. After 30 min, the mixture was concentrated under vacuum to afford 117 mg of the title compound as a yellow solid. ¹H NMR (DMSO-d₆) 0.98 (t, 3H, J = 7.3 Hz), 1.06 (d, 3H, J = 7.3 Hz), 1.94-2.04 (m, 2H), 2.39 (q, 2H, J = 7.3 Hz), 5.15 (q, 1H, J = 7.0 Hz), 7.57-7.81 (m, 11H), 8.11-8.19 (m, 2H), 8.56 (s, 1H), 8.71 (d, 1H, J = 4.3 Hz), 9.00 (s, 1H), 9.32-9.35 (m, 2H), 11.02 (s, 1H).

### Example 11 C

### (S)-7-[(4'-Trifluoromethyl-biphenyl-2-carbonyl)-amino]-quinoline-3-carboxylic acid (1-pyridin-2-yl-propyl)-amide

7-[(4'-Trifluoromethyl-biphenyl-2-carbonyl)-amino]-quinoline-3-carboxylic acid (150 mg, 0.34 mmol) was combined with 1-(2-pyridyl)-propylamine hydrochloride (59 mg, 0.34 mmol), 1-(3-dimethylaminopropyl)-3-ethylcarbodiimide hydrochloride (79 mg, 0.41 mmol), 1-hydroxybenzotriazole (51 mg, 0.38 mmol), and triethylamine (0.19 ml, 1.37 mmol) in 3 ml of dichloromethane. After stirring overnight at ambient temperature, the reaction mixture was diluted with 50 ml of dichloromethane, and the organic phase washed sequentially with water, and brine, and then dried over anhydrous magnesium sulfate, filtered and concentrated under vacuum to afford 175 mg of a colorless solid. This material was purified by column chromatography on silica gel eluting with 60-80% ethyl acetate in hexanes. The product-containing fractions were concentrated to obtain 78 mg of the title compound as a colorless solid. Optical Rotation: [α]_{D} = +75.6° (c = 0.40 mg/ml; CH30H). MS (APCI) 555 (M+1)⁺; 553 (M-1)⁻; ¹H NMR (DMSO-d₆) 0.91 (t, 3H, J = 7.4 Hz), 1.84-1.94 (m, 2H), 5.00-5.03 (m, 1H), 7.22 (ddd, 1H, J = 7.5, 4.8, 1.0 Hz), 7.42 (d, 1H, J = 7.9 Hz), 7.51-7.74 (m, 10H), 7.97 (d, 1H, J = 8.9 Hz), 8.38 (d, 1H, J = 1.7 Hz), 8.49 (ddd, 1H, J = 4.8, 1.9, 1.0 Hz), 8.75 (d, 1H, J = 1.7 Hz), 9.02 (d, 1H, J = 8.1 Hz), 9.20 (d, 1H, J = 2.3 Hz), 10.82 (s, 1H).

### Example 11 D

### (S)-7-[(4'-Trifluoromethyl-biphenyl-2-carbonyl)-amino]-quinoline-3-carboxylic acid (1-pyridin-2-yl-propyl)-amide ethanesulfonate

(S)-7-[(4'-Trifluoromethyl-biphenyl-2-carbonyl)-amino]-quinoline-3-carboxylic acid (1-pyridin-2-yl-propyl)-amide (67 mg) was dissolved in 5 ml of ethyl acetate, and ethanesulfonic acid (13 mg, 1 equivalent) was added as a solution in 1.5 ml of ethyl acetate. After 60 min, the mixture was concentrated under vacuum to afford 80 mg of the title compound as a yellow solid. ¹H NMR (DMSO-d₆) 0.98 (t, 3H, J = 7.3 Hz), 1.06 (d, 3H, J = 7.3 Hz), 1.94-2.04 (m, 2H), 2.39 (q, 2H, J = 7.3 Hz), 5.15 (q, 1H, J = 7.0 Hz), 7.57-7.81 (m, 11H), 8.11-8.19 (m, 2H), 8.56 (s, 1H), 8.71 (d, 1H, J = 4.3 Hz), 9.00 (s, 1H), 9.32-9.35 (m, 2H), 11.02 (s, 1H).

### Example 12

### 7-[(4'-Trifluoromethyl-biphenyl-2-carbonyl)-amino]-quinoline-3-carboxylic acid (pyridin-2-ylmethyl)-amide, ethanesulfonate

7-[(4'-Trifluoromethyl-biphenyl-2-carbonyl)-amino]-quinoline-3-carboxylic acid (100 mg, 0.23 mmol) was combined with 2-aminomethyl-pyridine (25 mg, 0.23 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was diluted with 50 ml of dichloromethane, and the organic phase washed sequentially with water, and brine, and then dried over anhydrous magnesium sulfate, filtered and concentrated under vacuum to provide a residue. This material was purified by preparative thin-layer chromatography on silica eluting with 4% methanol in dichloromethane. The product-containing band was collected and eluted with 5% methanol in ethyl acetate to afford 59 mg of product. This material was dissolved in 5 ml of ethyl acetate, and ethanesulfonic acid (12 mg, 1 equivalent) was added as a solution in 1 ml of diethyl ether. After 30 min, the mixture was concentrated under vacuum to afford 68 mg of the title compound as a yellow solid. MS (APCI) 527 (M+1)⁺; 525 (M-1)⁻; ¹H NMR (DMSO-d₆) 1.00 (t, 3H, J = 7.4 Hz), 2.34 (q, 2H, J = 7.4 Hz), 4.74 (d, 2H, J = 5.2 Hz), 7.51-7.74 (m, 11H), 8.05 (t, 1H, J = 9.1 Hz), 8.16 (t, 1H), 8.49 (s, 1H), 8.67 (d, 1H, J = 5.0 Hz), 8.90 (s, 1H), 9.29 (s, 1H), 9.57 (s, 1H), 10.95 (s, 1H).

### Example 13

### 7-[(4'-Trifluoromethyl-biphenyl-2-carbonyl)-amino]-quinoline-3-carboxylic acid (2-pyridin-2-yl-ethyl)-amide, ethanesulfonate

7-[(4'-Trifluoromethyl-biphenyl-2-carbonyl)-amino]-quinoline-3-carboxylic acid (100 mg, 0.23 mmol) was combined with 1-amino-2-(2-pyridyl)-ethane (28 mg, 0.23 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was diluted with 50 ml of dichloromethane, and the organic phase washed sequentially with water, and brine, and then dried over anhydrous magnesium sulfate, filtered and concentrated under vacuum to provide a residue. This material was purified by preparative thin-layer chromatography on silica eluting with 4% methanol in dichloromethane. The product-containing band was collected and eluted with 5% methanol in ethyl acetate to afford 65 mg of product. This material was dissolved in 5 ml of ethyl acetate, and ethanesulfonic acid (15 mg, 1 equivalent) was added as a solution in 1 ml of diethyl ether. After 30 min, the mixture was concentrated under vacuum to afford 88 mg of the title compound as a yellow solid. MS (APCI) 541 (M+1)⁺; 539 (M-1)⁻; ¹H NMR (DMSO-d₆) 1.02 (t, 3H, J = 7.5 Hz), 2.35 (q, 2H, J = 7.5 Hz), 3.21 (t, 2H, J = 6.2 Hz), 3.72 (m, 2H), 7.52-7.76 (m, 10H), 7.84 (d, 1H, J = 8.1 Hz), 7.99 (d, 1H, J = 8.7 Hz), 8.31 (t, 1H), 8.44 (s, 1H), 8.68 (s, 1H), 8.75 (d, 1H, J = 5.4 Hz), 8.93 (d, 1H, J = 5.4 Hz), 9.12 (s, 1H), 9.40 (s, 1H), 9.80 (d, 1H), 10.85 (s, 1H).

### Example 14

### 7-[(4'-Trifluoromethyl-biphenyl-2-carbonyl)-amino]-quinoline-3-carboxylic acid ethylamide, ethanesulfonate

7-[(4'-Trifluoromethyl-biphenyl-2-carbonyl)-amino]-quinoline-3-carboxylic acid (100 mg, 0.23 mmol) was combined with ethylamine hydrochloride (19 mg, 0.23 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was diluted with 50 ml of dichloromethane, and the organic phase washed sequentially with water, and brine, and then dried over anhydrous magnesium sulfate, filtered and concentrated under vacuum to provide a residue. This material was purified by preparative thin-layer chromatography on silica eluting with ethyl acetate The product-containing band was collected and eluted with 5% methanol in ethyl acetate to afford 52 mg of product. This material was dissolved in 5 ml of ethyl acetate, and ethanesulfonic acid (12 mg, 1 equivalent) was added as a solution in 1 ml of diethyl ether. After 30 min, the mixture was concentrated under vacuum to afford 61 mg of the title compound as a yellow solid. MS (APCI) 464 (M+1)⁺; 462 (M-1)⁻; ¹H NMR (DMSO-d₆) 1.06 (t, 3H, J = 7.4 Hz), 1.19 (t, 3H, J = 7.2 Hz), 2.41 (q, 2H, J = 7.4 Hz), 3.38 (qd, 2H, J = 7.2, 5.6 Hz), 7.57-7.82 (m, 9H), 8.19 (d, 1H, J = 8.9 Hz), 8.67 (s, 1H), 8.93 (t, 1H, J = 5.4 Hz), 9.10 (s, 1H), 9.37 (d, 1H, J = 2.0 Hz), 11.15 (s, 1H).

### Example 15

### 7-[(4'-Trifluoromethyl-biphenyl-2-carbonyl)-amino]-quinoline-3-carboxylic acid butylamide, ethanesulfonate

7-[(4'-Trifluoromethyl-biphenyl-2-carbonyl)-amino]-quinoline-3-carboxylic acid (100 mg, 0.23 mmol) was combined with butylamine (17 mg, 0.23 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was diluted with 50 ml of dichloromethane, and the organic phase washed sequentially with water, and brine, and then dried over anhydrous magnesium sulfate, filtered and concentrated under vacuum to provide a residue. This material was purified by preparative thin-layer chromatography on silica eluting with ethyl acetate. The product-containing band was collected and eluted with 5% methanol in ethyl acetate to afford 67 mg of product. This material was dissolved in 5 ml of ethyl acetate, and ethanesulfonic acid (15 mg, 1 equivalent) was added as a solution in 1 ml of diethyl ether. After 30 min, the mixture was concentrated under vacuum to afford 75 mg of the title compound as a yellow solid. MS (APCI) 492 (M+1)⁺; 490 (M-1)⁻ ; ¹H NMR (DMSO-d₆) 0.90 (t, 3H), 1.05 (t, 3H), 1.35 (tq, 2H), 1.55 (tt, 2H), 2.40 (q, 2H), 3.35 (dt, 2H), 7.55-7.80 (m, 9H), 8.20 (d, 1H), 8.65 (s, 1H), 8.85 (t, 1H), 9.07 (s, 1H), 9.35 (s, 1H), 11.15 (s, 1H).

### Example 16

### 7-[(4'-Trifluoromethyl-biphenyl-2-carbonyl)-amino]-quinoline-3-carboxylic acid (thiophen-2-ylmethyl)-amide, ethanesulfonate

7-[(4'-Trifluoromethyl-biphenyl-2-carbonyl)-amino]-quinoline-3-carboxylic acid (100 mg, 0.23 mmol) was combined with 2-aminomethyl-thiophen (26 mg, 0.23 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was diluted with 50 ml of dichloromethane, and the organic phase washed sequentially with water, and brine, and then dried over anhydrous magnesium sulfate, filtered and concentrated under vacuum to afford 92 mg of product. This material was dissolved in 10 ml of ethyl acetate, and ethanesulfonic acid (19 mg, 1 equivalent) was added as a solution in 1 ml of diethyl ether. After 30 min, the mixture was concentrated under vacuum to afford 103 mg of the title compound as a yellow solid. MS (APCI) 532 (M+1)⁺; 530 (M-1)⁻; ¹H NMR (DMSO-d₆) 1.06 (t, 3H, J = 7.4 Hz), 2.41 (q, 2H, J = 7.4 Hz), 4.72 (d, 2H, J = 5.6 Hz), 7.10 (dd, 1H, J = 3.3, 1.0 Hz), 7.43 (dd, 1H, J = 4.9, 1.3 Hz), 7.56-7.83 (m, 9H), 8.17 (d, 1H, J = 9.8 Hz), 8.64 (s, 1H), 9.09 (s, 1H), 9.38 (d, 1H, J = 2.0), 9.59 (t, 1H, J = 5.6 Hz).

### Example 17

### 7-[(4'-Trifluoromethyl-biphenyl-2-carbonyl)-amino]-quinoline-3-carboxylic acid (1-methyl-1-pyridin-2-yl-ethyl)-amide

7-[(4'-Trifluoromethyl-biphenyl-2-carbonyl)-amino]-quinoline-3-carboxylic acid (100 mg, 0.23 mmol) was combined with 2-amino-2-(2-pyridyl)-propane (47 mg, 0.35 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was diluted with 50 ml of dichloromethane, and the organic phase washed sequentially with water, and brine, and then dried over anhydrous magnesium sulfate, filtered and concentrated under vacuum to provide a residue. This material was purified by preparative thin-layer chromatography on silica eluting with 4% methanol in dichloromethane. The product-containing band was collected and eluted with 5% methanol in ethyl acetate to afford 50 mg of the title compound as a yellow solid. MS (APCI) 555 (M+1)⁺; 553 (M-1)⁻; ¹H NMR (DMSO-d₆) 1.72 (s, 6H), 7.22 (dd, 1H, J = 7.3, 4.6 Hz), 7.49 (d, 1H, J = 8.2 Hz), 7.58 (t, 1H, J = 7.6 Hz), 7.63-7.78 (m, 9H), 8.02 (d, 1H, J = 9.2 Hz), 8.42 (s, 1H), 8.51 (d, 1H, J = 4.6 Hz), 8.76 (d, 1H, J = 2.0 Hz), 8.89 (s, 1H), 9.18 (d, 1H, J = 2.0 Hz), 10.90 (s, 1H).

### Example 18

### (S)-7-[(4'-Trifluoromethyl-biphenyl-2-carbonyl)-amino]-quinoline-3-carboxylic acid (1-pyridin-2-yl-ethyl)-amide

7-[(4'-Trifluoromethyl-biphenyl-2-carbonyl)-amino]-quinoline-3-carboxylic acid (100 mg, 0.23 mmol) was combined with (S)-1-amino-1-(2-pyridyl)-ethane (51 mg, 0.41 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was diluted with 50 ml of dichloromethane, and the organic phase washed sequentially with water, and brine, and then dried over anhydrous magnesium sulfate, filtered and concentrated under vacuum to provide a residue. This material was purified by preparative thin-layer chromatography on silica eluting with ethyl acetate. The product-containing band was collected and eluted with 5% methanol in ethyl acetate to afford 76 mg of the title compound. Optical Rotation: [α]_{D} = +63.9° (c = 0.39 mg/ml; CH30H); MS (APCI) 541 (M+1)⁺; 539 (M-1)⁻; ¹H NMR (DMSO-d₆) 1.52 (d, 3H, J = 6.9 Hz), 5.22 (dq, 1H), 7.24 (t, 1H, J = 5.9 Hz), 7.43 (d, 1H, J = 7.7 Hz), 7.52-7.76 (m, 10H), 7.98 (d, 1H, J = 8.6 Hz), 8.40 (s, 1H), 8.51 (d, 1H, J = 5.0 Hz), 8.77 (s, 1H), 9.11 (d, 1H, J = 7.3 Hz), 9.22 (s, 1H), 10.81 (s, 1H).

### Example 18A

### (R)-7-[(4'-Trifluoromethyl-biphenyl-2-carbonyl)-amino]-quinoline-3-carboxylic acid (1-pyridin-2-yl-ethyl)-amide ethanesulfonate

7-[(4'-Trifluoromethyl-biphenyl-2-carbonyl)-amino]-quinoline-3-carboxylic acid (100 mg, 0.23 mmol) was combined with (R)-1-(2-pyridyl)-ethylamine (50 mg, 0.41 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was diluted with 50 ml of dichloromethane, and the organic phase washed sequentially with water, and brine, and then dried over anhydrous magnesium sulfate, filtered and concentrated under vacuum to provide a residue. This material was purified by preparative thin-layer chromatography on silica eluting with ethyl acetate. The product-containing band was collected and eluted with 5% methanol in ethyl acetate to afford 50 mg of the title compound as a colorless solid. Optical Rotation: [α]_{D} = -86.3° (c = 0.39 mg/ml; CH30H). A portion of this material (40 mg) was dissolved in 5 ml of ethyl acetate, and ethanesulfonic acid (7.5 mg, 1 equivalent) was added as a solution in 1 ml of diethyl ether. After 30 min, the mixture was concentrated under vacuum to afford 38 mg of the title compound as a yellow solid. MS (APCI) 541 (M+1)⁺; 539 (M-1)⁻; ¹H NMR (DMSO-d₆) 1.05 (t, 3H, J = 7.4 Hz), 1.62 (d, 3H, J = 6.9 Hz), 2.40 (q, 2H, J = 7.4 Hz), 5.34 (m, 1H), 7.56-7.87 (m, 11H), 8.14 (d, 1H, J = 8.9 Hz), 8.23 (m, 1H), 8.59 (s, 1H), 8.73 (d, 1H, J = 4.3 Hz), 9.04 (s, 1H), 9.35 (s, 1H), 9.45 (d, 1H,J =5.9 Hz), 11.03 (s, 1H).

### Example 19

### 7-[(4'-Trifluoromethyl-biphenyl-2-carbonylfamino]-quinoline-3-carboxylic acid amide

7-[(4'-Trifluoromethyl-biphenyl-2-carbonyl)-amino]-quinoline-3-carboxylic acid (300 mg, 0.69 mmol) was combined with ammonium chloride (55 mg, 1.04 mmol), 1-(3-dimethylaminopropyl)-3-ethylcarbodiimide hydrochloride (159 mg, 0.83 mmol), 1hydroxybenzotriazole (103 mg, 0.76 mmol), and triethylamine (0.38 ml, 2.76 mmol) in 5 ml of dichloromethane. After stirring overnight at ambient temperature, the reaction mixture was concentrated, the residue was suspended in water, and the solid was collected by vacuum filtration to afford 219 mg of the title compound as an off-white solid. MS (APCI) 436 (M+1)⁺; 434 (M-1)⁻ ; ¹H NMR (DMSO-d₆) 7.51-7.72 (m, 10H), 7.93 (d, 1H, J = 8.7 Hz), 8.21 (s, 1H), 8.38 (s, 1H), 8.70 (s, 1H), 9.19 (s, 1H), 10.80 (s, 1H).

### Example 20

### 7-[(4'-Trifluoromethyl-biphenyl-2-carbonyl)-amino]-quinoline-3-carboxylic acid benzylamide

7-[(4'-Trifluoromethyl-biphenyl-2-carbonyl)-amino]-quinoline-3-carboxylic acid (40 mg, 0.092 mmol) was combined with benzylamine (15 mg, 0.14 mmol), 1-(3-dimethylaminopropyl)-3-ethylcarbodiimide hydrochloride (21 mg, 0.11 mmol), 1-hydroxybenzotriazole (14 mg, 0.10 mmol), and triethylamine (0.051 ml, 0.37 mmol) in 1.5 ml of dichloromethane. After stirring overnight at ambient temperature, the reaction mixture was diluted with 50 ml of dichloromethane, and the organic phase washed sequentially with water, and brine, and then dried over anhydrous magnesium sulfate, filtered and concentrated under vacuum to provide a residue. This material was purified by preparative thin-layer chromatography on silica eluting with 4% methanol in dichloromethane. The product-containing band was collected and eluted with 5% methanol in ethyl acetate to afford 20 mg of the title compound as a colorless solid. MS (APCI) 526 (M+1)⁺; 524 (M-1)⁻; ¹H NMR (DMSO-d₆) 4.55 (d, 2H, J = 5.9 Hz), 7.24-7.39 (m, 5H), 7.56-7.77 (m, 9H), 8.00 (d, 1H, J = 8.9 Hz), 8.43 (s, 1H), 8.76 (d, 1H, J = 2.0 Hz), 9.25 (d, 1H, J = 2.3 Hz), 9.34 (t, 1H, J = 5.9 Hz), 10.85 (s, 1H).

### Example 21

### 7-[(4'-Trifluoromethyl-biphenyl-2-carbonyl)-amino]-quinoline-3-carboxylic acid 4-methoxy-benzylamide

7-[(4'-Trifluoromethyl-biphenyl-2-carbonyl)-amino]-quinoline-3-carboxylic acid (40 mg, 0.092 mmol) was combined with 4-methoxybenzylamine (15 mg, 0.14 mmol), 1-(3-dimethylaminopropyl)-3-ethylcarbodiimide hydrochloride (21 mg, 0.11 mmol), 1-hydroxybenzotriazole (14 mg, 0.10 mmol), and triethylamine (0.051 ml, 0.37 mmol) in 1.5 ml of dichloromethane. After stirring overnight at ambient temperature, the reaction mixture was diluted with 50 ml of dichloromethane, and the organic phase washed sequentially with water, and brine, and then dried over anhydrous magnesium sulfate, filtered and concentrated under vacuum to provide a residue. This material was purified by preparative thin-layer chromatography on silica eluting with 4% methanol in dichloromethane. The product-containing band was collected and eluted with 5% methanol in ethyl acetate to afford 9 mg of the title compound as a colorless solid. MS (APCI) 556 (M+1)⁺; 554 (M-1)⁻; ¹H NMR (DMSO-d₆) 3.73 (s, 3H), 4.47 (d, 2H, J = 5.9 Hz), 6.91 (d, 2H, J = 8.6 Hz), 7.30 (d, 2H, J = 8.6 Hz), 7.56-7.77 (m, 9H), 8.00 (d, 1H, J = 8.9 Hz), 8.43 (s, 1H), 8.74 (d, 1H, J = 2.0 Hz), 9.24-9.26 (m, 2H), 10.85 (S, 1H).

### Example 22

### 7-[(4'-Trifluoromethyl-biphenyl-2-carbonyl)-amino]-quinoline-3-carboxylic acid 4-chloro-benzylamide

7-[(4'-Trifluoromethyl-biphenyl-2-carbonyl)-amino]-quinoline-3-carboxylic acid (40 mg, 0.092 mmol) was combined with 4-chlorobenzylamine (19 mg, 0.14 mmol), 1-(3-dimethylaminopropyl)-3-ethylcarbodiimide hydrochloride (21 mg, 0.11 mmol), 1-hydroxybenzotriazole (14 mg, 0.10 mmol), and triethylamine (0.051 ml, 0.37 mmol) in 1.5 ml of dichloromethane. After stirring overnight at ambient temperature, the reaction mixture was concentrated, the residue was suspended in water, and the solid was collected by vacuum filtration. This material was then slurried in a 1:1 methanol-dichloromethane solution, and the solid collected by vacuum filtration to afford the title compound as a colorless solid. MS (APCI) 560 & 562 (M+1)⁺; 558 & 560 (M-1)⁻; ¹H NMR (DMSO-d₆) 4.53 (d, 2H, J = 5.6 Hz), 7.40 (s, 4H), 7.55-7.77 (m, 9H), 8.00 (d, 1H, J = 8.9 Hz), 8.43 (s, 1H), 8.76 (d, 1H, J = 1.6 Hz), 9.25 (d, 1H, J = 2.0 Hz), 9.36 (t, 1H, J = 5.9 Hz), 10.85 (s, 1H).

### Example 23

### 7-[(4'-Trifluoromethyl-biphenyl-2-carbonyl)-amino]-quinoline-3-carboxylic acid 4-methyl-benzylamide

7-[(4'-Trifluoromethyl-biphenyl-2-carbonyl)-amino]-quinoline-3-carboxylic acid (40 mg, 0.092 mmol) was combined with 4-methylbenzylamine (17 mg, 0.14 mmol), 1-(3-dimethylaminopropyl)-3-ethylcarbodiimide hydrochloride (21 mg, 0.11 mmol), 1-hydroxybenzotriazole (14 mg, 0.10 mmol), and triethylamine (0.051 ml, 0.37 mmol) in 1.5 ml of dichloromethane. After stirring overnight at ambient temperature, the reaction mixture was diluted with 50 ml of dichloromethane, and the organic phase washed sequentially with water, and brine, and then dried over anhydrous magnesium sulfate, filtered and concentrated under vacuum to provide a residue. This material was purified by preparative thin-layer chromatography on silica eluting with 4% methanol in dichloromethane. The product-containing band was collected and eluted with 5% methanol in ethyl acetate to afford 20 mg of the title compound as a colorless solid. MS (APCI) 540 (M+1)⁺; 538 (M-1)⁻; ¹H NMR (DMSO-d₆) 2.25 (s, 3H), 4.46 (d, 2H, J = 5.8 Hz), 7.12 (d, 2H, J = 8.0 Hz), 7.22 (d, 2H, J = 8.0 Hz), 7.53-7.73 (m, 9H), 7.96 (d, 1H, J = 8.9 Hz), 8.40 (s, 1H), 8.72 (d, 1H, J = 2.1 Hz), 9.21 (d, 1H, J = 2.3 Hz), 9.25 (t, 1H, J = 5.9 Hz), 10.82 (s, 1H).

### Example 24

### 7-[(4'-Trifluoromethyl-biphenyl-2-carbonyl)-amino]-quinoline-3-carboxylic acid cyclopropylmethyl-amide

7-[(4'-Trifluoromethyl-biphenyl-2-carbonyl)-amino]-quinoline-3-carboxylic acid (100 mg, 0.23 mmol) was combined with cyclopropylmethylamine (29 mg, 0.27 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was diluted with 50 ml of dichloromethane, and the organic phase washed sequentially with water, and brine, and then dried over anhydrous magnesium sulfate, filtered and concentrated under vacuum to afford 101 mg of the title compound as a colorless solid. MS (APCI) 490 (M+1)⁺; 488 (M-1)⁻; ¹H NMR (DMSO-d₆) 0.24-0.29 (m, 2H), 0.44-0.50 (m, 2H), 1.03-1.09 (m, 1H), 3.21 (t, 2H, J = 6.1 Hz), 7.55-7.77 (m, 9H), 8.00 (d, 1H, J = 8.9 Hz), 8.43 (d, 1H, J = 1.7 Hz), 8.72 (d, 1H, J = 2.0 Hz), 8.87 (t, 1H, J = 5.6 Hz), 9.22 (d, 1H, J = 2.3 Hz), 10.85 (s, 1H).

### Example 25

### 7-[(4'-Trifluoromethyl-biphenyl-2-carbonyl)-amino]-quinoline-3-carboxylic acid 4-fluoro-benzylamide

7-[(4'-Trifluoromethyl-biphenyl-2-carbonyl)-amino]-quinoline-3-carboxylic acid (100 mg, 0.23 mmol) was combined with 4-fluoromethylamine (34 mg, 0.27 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was concentrated, the residue was suspended in water, and the solid was collected by vacuum filtration to afford 96 mg of the title compound as a colorless solid. MS (APCI) 544 (M+1)⁺; 542 (M-1)⁻ ; ¹H NMR (DMSO-d₆) 4.53 (d, 2H, J = 5.9 Hz), 7.14-7.20 (m, 2H), 7.39-7.44 (m, 2H), 7.55-7.77 (m, 9H), 8.00 (d, 1H, J = 8.9 Hz), 8.43 (d, 1H, J = 1.6 Hz), 8.76 (d, 1H, J = 1.6 Hz), 9.25 (d, 1H, J = 2.0 Hz), 9.34 (t, 1H, J = 5.9 Hz), 10.85 (s, 1H).

### Example 26

### 7-[(4'-Trifluoromethyl-biphenyl-2-carbonyl)-amino]-quinoline-3-carboxylic acid isopropyl-amide

7-[(4'-Trifluoromethyl-biphenyl-2-carbonyl)-amino]-quinoline-3-carboxylic acid (100 mg, 0.23 mmol) was combined with isopropylamine (16 mg, 0.27 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature the reaction mixture was diluted with 50 ml of dichloromethane, and the organic phase washed sequentially with water, and brine, and then dried over anhydrous magnesium sulfate, filtered and concentrated under vacuum to afford 96 mg of the title compound as a yellow solid. MS (APCI) 478 (M+1)⁺; 476 (M-1)⁻; ¹H NMR (DMSO-d₆) 1.21 (d, 6H, J = 6.6 Hz), 4.10-4.20 (m, 1H), 7.56-7.77 (m, 9H), 7.99 (d, 1H, J = 9.2 Hz), 8.42 (d, 1H, J = 1.7 Hz), 8.54 (d, 1H, J = 7.6 Hz), 8.70 (d, 1H, J = 1.6 Hz), 9.20 (d, 1H, J = 2.0 Hz), 10.85 (s, 1H).

### Example 27

### 7-[(4'-Trifluoromethyl-biphenyl-2-carbonyl)-amino]-quinoline-3-carboxylic acid benzhydryl-amide

7-[(4'-Trifluoromethyl-biphenyl-2-carbonyl)-amino]-quinoline-3-carboxylic acid (100 mg, 0.23 mmol) was combined with aminodiphenylmethane (42 mg, 0.23 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was diluted with 50 ml of dichloromethane, and the organic phase washed sequentially with water, and brine, and then dried over anhydrous magnesium sulfate, filtered and concentrated under vacuum to provide a residue. This material was purified by preparative thin-layer chromatography on silica eluting with ethyl acetate. The product-containing band was collected and eluted with 5% methanol in ethyl acetate to afford 66 mg of the title compound as a colorless solid. MS (APCI) 602 (M+1)⁺; 600 (M-1)⁻ ; ¹H NMR (DMSO-d₆) 6.47 (d, 1H, J = 8.6 Hz), 7.26-7.43 (m, 10H), 7.56-7.77 (m, 9H), 8.00 (d, 1H, J = 8.9 Hz), 8.44 (s, 1H), 8.83 (d, 1H, J = 2.0 Hz), 9.25 (d, 1H, J = 3.3 Hz), 9.58 (d, 1H, J = 8.6 Hz), 10.85 (s, 1H).

### Example 28

### 7-[(4'-Trifluoromethyl-biphenyl-2-carbonyl)-amino]-quinoline-3-carboxylic acid cyclopropylamide

7-[(4'-Trifluoromethyl-biphenyl-2-carbonyl)-amino]-quinoline-3-carboxylic acid (100 mg, 0.23 mmol) was combined with cyclopropylamine (20 mg, 0.35 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was diluted with 50 ml of dichloromethane, and the organic phase washed sequentially with water, and brine, and then dried over anhydrous magnesium sulfate, filtered and concentrated under vacuum to afford the title compound. MS (APCI) 476 (M+1)⁺; 474 (M-1)⁻; ¹H NMR (DMSO-d₆) 0.57-0.59 (m, 2H), 0.67-0.72 (m, 2H), 2.85-2.88 (m, 1H), 7.51-7.72 (m, 9H), 7.94 (d, 1H, J = 8.9 Hz), 8.37 (s, 1H), 8.62 (s, 1H), 8.69 (d, 1H, J = 4.2 Hz), 9.13 (s, 1H), 10.80 (s, 1H).

### Example 29

### 7-[(4'-Trifluoromethyl-biphenyl-2-carbonyl)-amino]-quinoline-3-carboxylic acid [1-(4-fluoro-phenyl)-ethyl]-amide

7-[(4'-Trifluoromethyl-biphenyl-2-carbonyl)-amino]-quinoline-3-carboxylic acid (100 mg, 0.23 mmol) was combined with 1-(4-fluorophenyl)-ethylamine (32 mg, 0.23 mmol), 1-(3-dimethylaminopropyl)-3-ethylcarbodiimide hydrochloride (53 mg, 0.27 mmol), 1-hydroxybenzotriazole (34 mg, 0.25 mmol), and triethylamine (0.13 ml, 0.92 mmol) in 1.5 ml of dichloromethane. After stirring overnight at ambient temperature, the reaction mixture was diluted with 50 ml of ethyl acetate, and the organic phase washed sequentially with water, and brine, and then dried over anhydrous magnesium sulfate, filtered and concentrated under vacuum to afford 120 mg of the title compound as a yellow solid. MS (APCI) 558 (M+1)⁺; 556 (M-1)⁻ ; ¹H NMR (DMSO-d₆) 1.50 (d, 3H, J = 6.9 Hz), 5.22 (quintet, 1H, J = 6.9), 7.13-7.20 (m, 2H), 7.43-7.52 (m, 2H), 7.55-7.82 (m, 9H), 8.01 (d, 1H, J = 9.2 Hz), 8.43 (d, 1H, J = 1.6 Hz), 8.75 (d, 1H, J = 1.6 Hz), 9.11 (d, 1H, J = 7.6 Hz), 9.22 (d, 1H, J = 2.3 Hz), 10.85 (s, 1H).

### Example 30

### 7-[(4'-Trifluoromethyl-biphenyl-2-carbonyl)-amino]-quinoline-3-carboxylic acid 3-methyl-benzylamide

7-[(4'-Trifluoromethyl-biphenyl-2-carbonyl)-amino]-quinoline-3-carboxylic acid (50 mg, 0.11 mmol) was combined with 3-methylbenzylamine (14 mg, 0.11 mmol), 1-(3-dimethylaminopropyl)-3-ethylcarbodiimide hydrochloride (26 mg, 0.14 mmol), 1-hydroxybenzotriazole (17 mg, 0.13 mmol), and triethylamine (0.064 ml, 0.46 mmol) in 1 ml of dichloromethane. After stirring overnight at ambient temperature, the reaction mixture was diluted with 30 ml of ethyl acetate, and the organic phase washed sequentially with water, and brine, and then dried over anhydrous magnesium sulfate, filtered and concentrated under vacuum to afford 52 mg of the title compound as a colorless solid. MS (APCI) 540 (M+1)⁺; 538 (M-1)⁻; ¹H NMR (DMSO-d₆) 2.29 (s, 3H), 4.51 (d, 2H, J = 7.0 Hz), 7.06-7.25 (m, 4H), 7.55-7.77 (m, 9H), 8.00 (d, 1H, J = 8.9 Hz), 8.43 (d, 1H, J = 2.0 Hz), 8.76 (d, 1H, J = 2.0 Hz), 9.25 (d, 1H, J = 2.0 Hz), 9.30 (t, 1H, J= 5.9 Hz), 10.85 (s, 1H).

### Example 31

### 7-[(4'-Trifluoromethyl-biphenyl-2-carbonyl)-amino]-quinoline-3-carboxylic acid 3-methoxy-benzylamide

7-[(4'-Trifluoromethyl-biphenyl-2-carbonyl)-amino]-quinoline-3-carboxylic acid (50 mg, 0.11 mmol) was combined with 3-methoxybenzylamine (16 mg, 0.11 mmol), 1-(3-dimethylaminopropyl)-3-ethylcarbodiimide hydrochloride (26 mg, 0.14 mmol), 1-hydroxybenzotriazole (17 mg, 0.13 mmol), and triethylamine (0.064 ml, 0.46 mmol) in 1 ml of dichloromethane. After stirring overnight at ambient temperature, the reaction mixture was diluted with 30 ml of ethyl acetate, and the organic phase washed sequentially with water, and brine, and then dried over anhydrous magnesium sulfate, filtered and concentrated under vacuum to afford 53 mg of the title compound as a colorless solid. MS (APCI) 556 (M+1)⁺; 554 (M-1)⁻; ¹H NMR (DMSO-d₆) 3.74 (s, 3H), 4.51 (d, 2H, J = 5.6 Hz), 6.83 (dd, 1H, J = 7.4, 2.4 Hz), 6.93-6.95 (m, 2H), 7.26 (t, 1H, J = 8.3), 7.55-7.77 (m, 9H), 7.99 (d, 1H, J = 8.9 Hz), 8.42 (s, 1H), 8.76 (d, 1H, J = 2.3 Hz), 9.25 (d, 1H, J = 2.3 Hz), 9.31 (t, 1H, J = 5.9), 10.83 (s, 1H).

### Example 32

### 7-[(4'-Trifluoromethyl-biphenyl-2-carbonyl)-amino]-quinoline-3-carboxylic acid 3-chloro-benzylamide

7-[(4'-Trifluoromethyl-biphenyl-2-carbonyl)-amino]-quinoline-3-carboxylic acid (50 mg, 0.11 mmol) was combined with 3-chlorobenzylamine (16 mg, 0.11 mmol), 1-(3-dimethylaminopropyl)-3-ethylcarbodiimide hydrochloride (26 mg, 0.14 mmol), 1-hydroxybenzotriazole (17 mg, 0.13 mmol), and triethylamine (0.064 ml, 0.46 mmol) in 1 ml of dichloromethane. After stirring overnight at ambient temperature, the reaction mixture was diluted with 30 ml of ethyl acetate, and the organic phase washed sequentially with water, and brine, and then dried over anhydrous magnesium sulfate, filtered and concentrated under vacuum to afford 56 mg of the title compound as a colorless solid. MS (APCI) 560 & 562 (M+1)⁺; 558 & 560 (M-1)⁻ ; ¹H NMR (DMSO-d₆) 4.54 (d, 2H, J = 5.9 Hz), 7.30-7.43 (m, 4H), 7.52-7.77 (m, 9H), 8.01 (d, 1H, J = 8.9 Hz), 8.43 (d, 1H, J = 1.6 Hz), 8.76 (d, 1H, J = 2.0 Hz), 9.25 (d, 1H, J = 2.0 Hz), 9.37 (t, 1H, J=6.0 Hz), 10.85 (S, 1H).

### Example 33

### 7-[(4'-Trifluoromethyl-biphenyl-2-carbonyl)-amino]-quinoline-3-carboxylic acid 2-fluoro-benzylamide

7-[(4'-Trifluoromethyl-biphenyl-2-carbonyl)-amino]-quinoline-3-carboxylic acid (50 mg, 0.11 mmol) was combined with 2-fluorobenzylamine (14mg, 0.11 mmol), 1-(3-dimethylaminopropyl)-3-ethylcarbodiimide hydrochloride (26 mg, 0.14 mmol), 1-hydroxybenzotriazole (17 mg, 0.13 mmol), and triethylamine (0.064 ml, 0.46 mmol) in 1 ml of dichloromethane. After stirring overnight at ambient temperature, the reaction mixture was diluted with 30 ml of ethyl acetate, and the organic phase washed sequentially with water, and brine, and then dried over anhydrous magnesium sulfate, filtered and concentrated under vacuum to afford 48 mg of the title compound as a colorless solid. MS (APCI) 544 (M+1)⁺; 542 (M-1)⁻; ¹H NMR (DMSO-d₆) 4.58 (d, 2H, J = 5.6 Hz), 7.16-7.23 (m, 2H), 7.29-7.36 (m, 1H), 7.42-7.47 (m, 1H), 7.55-7.77 (m, 9H), 7.99 (d, 1H, J = 8.8 Hz), 8.43 (s, 1H), 8.76 (d, 1H, J = 2.3 Hz), 9.25 (d, 1H, J = 2.3 Hz), 9.31 (t, 1H, J = 5.6 Hz), 10.85 (s, 1H).

### Example 34

### 7-[(4'-Trifluoromethyl-biphenyl-2-carbonyl)-amino]-quinoline-3-carboxylic acid 3-fluoro-benzylamide

7-[(4'-Trifluoromethyl-biphenyl-2-carbonyl)-amino]-quinoline-3-carboxylic acid (50 mg, 0.11 mmol) was combined with 3-fluorobenzylamine (14 mg, 0.11 mmol), 1-(3-dimethylaminopropyl)-3-ethylcarbodiimide hydrochloride (26 mg, 0.14 mmol), 1-hydroxybenzotriazole (17 mg, 0.13 mmol), and triethylamine (0.064 ml, 0.46 mmol) in 1 ml of dichloromethane. After stirring overnight at ambient temperature, the reaction mixture was diluted with 30 ml of ethyl acetate, and the organic phase washed sequentially with water, and brine, and then dried over anhydrous magnesium sulfate, filtered and concentrated under vacuum to afford 49 mg of the title compound as a colorless solid. MS (APCI) 544 (M+1)⁺; 542 (M-1)⁻; ¹H NMR (DMSO-d₆) 4.55 (d, 2H, J = 5.9 Hz), 7.08 (td, 1H, J = 8.3, 2.3 Hz), 7.17-7.23 (m, 2H), 7.35-7.43 (m, 1H), 7.55-7.77 (m, 9H), 8.00 (d, 1H, J = 8.9 Hz), 8.43 (s, 1H), 8.77 (d, 1H, J = 2.0 Hz), 9.25 (d, 1H, J = 2.0 Hz), 9.36 (t, 1H, J = 5.9 Hz), 10.85 (s, 1H).

### Example 35

### 7-[(4'-Trifluoromethyl-biphenyl-2-carbonyl)-amino]-quinoline-3-carboxylic acid 2-methyl-benzylamide

7-[(4'-Trifluoromethyl-biphenyl-2-carbonyl)-amino]-quinoline-3-carboxylic acid (50 mg, 0.11 mmol) was combined with 2-methylbenzylamine (14 mg, 0.11 mmol), 1-(3-dimethylaminopropyl)-3-ethylcarbodiimide hydrochloride (26 mg, 0.14 mmol), 1-hydroxybenzotriazole (17 mg, 0.13 mmol), and triethylamine (0.064 ml, 0.46 mmol) in 1 ml of dichloromethane. After stirring overnight at ambient temperature, the reaction mixture was concentrated, the residue was suspended in water, and the solid was collected by vacuum filtration to afford 50 mg of the title compound as a colorless solid. MS (APCI) 540 (M+1)⁺; 538 (M-1)⁻ ; ¹H NMR (DMSO-d₆) 2.35 (s, 3H), 4.52 (d, 2H, J = 5.6 Hz), 7.15-7.20 (m, 3H), 7.29-7.32 (m, 1H), 7.55-7.77 (m, 9H), 7.99 (d, 1H, J = 8.9 Hz), 8.43 (s, 1H), 8.77 (d, 1H, J = 2.0 Hz), 9.18 (t, 1H, J = 5.9 Hz), 9.25 (d, 1H, J=2.3 Hz), 10.83 (s, 1H).

### Example 36

### 7-[(4'-Trifluoromethyl-biphenyl-2-carbonyl)-amino]-quinoline-3-carboxylic acid 2-methoxy-benzylamide

7-[(4'-Trifluoromethyl-biphenyl-2-carbonyl)-amino]-quinoline-3-carboxylic acid (50 mg, 0.11 mmol) was combined with 2-methoxybenzylamine (16 mg, 0.11 mmol), 1-(3-dimethylaminopropyl)-3-ethylcarbodiimide hydrochloride (26 mg, 0.14 mmol), 1-hydroxybenzotriazole (17 mg, 0.13 mmol), and triethylamine (0.064 ml, 0.46 mmol) in 1 ml of dichloromethane. After stirring overnight at ambient temperature, the reaction mixture was diluted with 30 ml of ethyl acetate, and the organic phase washed sequentially with water, and brine, and then dried over anhydrous magnesium sulfate, filtered and concentrated under vacuum to afford 46 mg of the title compound as a colorless solid. MS (APCI) 556 (M+1)⁺; 554 (M-1)⁻; ¹H NMR (DMSO-d₆) 3.84 (s, 3H), 4.51 (d, 2H, J = 5.6 Hz), 6.92 (td, 1H, J = 7.6, 1.0 Hz), 7.01 (d, 1H, J = 7.6 Hz), 7.23-7.27 (m, 2H), 7.55-7.77 (m, 9H), 8.00 (d, 1H, J = 8.9 Hz), 8.43 (d, 1H, J = 1.6 Hz), 8.78 (d, 1H, J = 2.0 Hz), 9.14 (t, 1H, J = 5.8 Hz), 9.26 (d, 1H, J = 2.3 Hz), 10.85 (s, 1H).

### Example 37

### 7-[(4'-Trifluoromethyl-biphenyl-2-carbonyl)-amino]-quinoline-3-carboxylic acid 2-chloro-benzylamide

7-[(4'-Trifluoromethyl-biphenyl-2-carbonyl)-amino]-quinoline-3-carboxylic acid (50 mg, 0.11 mmol) was combined with 2-chlorobenzylamine (16 mg, 0.11 mmol), 1-(3-dimethylaminopropyl)-3-ethylcarbodiimide hydrochloride (26 mg, 0.14 mmol), 1-hydroxybenzotriazole (17 mg, 0.13 mmol), and triethylamine (0.064 ml, 0.46 mmol) in 1 ml of dichloromethane. After stirring overnight at ambient temperature, the reaction mixture was concentrated, the residue was suspended in water, and the solid was collected by vacuum filtration to afford 48 mg of the title compound as a colorless solid. MS (APCI) 558 & 560 (M-1)⁻; ¹H NMR (DMSO-d₆) 4.58 (d, 2H, J = 5.6 Hz), 7.27-7.37 (m, 2H), 7.41-7.46 (m, 2H), 7.53-7.74 (m, 9H), 7.99 (d, 1H, J = 8.9 Hz), 8.41 (s, 1H), 8.77 (s, 1H), 9.24 (d, 1H, J = 1.9 Hz), 9.31 (t, 1H, J = 5.6 Hz), 10.83 (s, 1H).

### Example 38

### 4'-Trifluoromethyl-biphenyl-2-carboxylic acid [3-(pyrrolidine-1-carbonyl)-quinolin-7-yl]-amide

7-[(4'-Trifluoromethyl-biphenyl-2-carbonyl)-amino]-quinoline-3-carboxylic acid (50 mg, 0.11 mmol) was combined with pyrrolidine (9 mg, 0.11 mmol), 1-(3-dimethylaminopropyl)-3-ethylcarbodiimide hydrochloride (26 mg, 0.14 mmol), 1-hydroxybenzotriazole (17 mg, 0.13 mmol), and triethylamine (0.064 ml, 0.46 mmol) in 1 ml of dichloromethane and allowed to react overnight The reaction mixture was concentrated, the residue was suspended in water, and the solid was collected by vacuum filtration. This material was purified by preparative thin-layer chromatography on silica eluting with ethyl acetate. The product-containing band was collected and eluted with 5% methanol in ethyl acetate to afford 26 mg of the title compound as a colorless solid. MS (APCI) 490 (M+1)⁺; 488 (M-1)⁻; ¹H NMR (DMSO-d₆) 1.82-1.92 (m, 4H), 3.52 (t, 4H, J = 6.3 Hz), 7.55-7.77 (m, 9H), 7.94 (d, 1H, J = 8.9 Hz), 8.40 (s, 1H), 8.47 (d, 1H, J = 1.7 Hz), 8.93 (d, 1H, J = 1.3 Hz), 10.80 (s, 1H).

### Example 39

### 4'-Trifluoromethyl-biphenyl-2-carboxylic acid [3-(morpholine-4-carbonyl)-quinolin-7-yl]-amide

7-[(4'-Trifluoromethyl-biphenyl-2-carbonyl)-amino]-quinoline-3-carboxylic acid (50 mg, 0.11 mmol) was combined with morpholine (11 mg, 0.12 mmol), 1-(3-dimethylaminopropyl)-3-ethylcarbodiimide hydrochloride (26 mg, 0.14 mmol), 1-hydroxybenzotriazole (17 mg, 0.13 mmol), and triethylamine (0.064 ml, 0.46 mmol) in 1 ml of dichloromethane and allowed to react overnight. The reaction mixture was concentrated, the residue was suspended in water, and the solid was collected by vacuum filtration. This material was purified by preparative thin-layer chromatography on silica eluting with ethyl acetate. The product-containing band was collected and eluted with 5% methanol in ethyl acetate to afford 28 mg of the title compound as a colorless solid. MS (APCI) 506 (M+1)⁺; 504 (M-1)⁻; ¹H NMR (DMSO-d₆) 3.40-3.75 (m, 8H), 7.55-7.76 (m, 9H), 7.96 (d, 1H, J = 8.9 Hz), 8.36 (d, 1H, J = 2.0 Hz), 8.41 (d, 1H, J = 1.7 Hz), 8.85 (d, 1H, J = 2.3 Hz), 10.80 (s, 1H).

### Example 40

### 7-[(4'-Trifluoromethyl-biphenyl-2-carbonyl)-amino]-quinoline-3-carboxylic acid diethylamide

7-[(4'-Trifluoromethyl-biphenyl-2-carbonyl)-amino]-quinoline-3-carboxylic acid (50 mg, 0.11 mmol) was combined with diethylamine hydrochloride (15 mg, 0.14 mmol), 1-(3-dimethylaminopropyl)-3-ethylcarbodiimide hydrochloride (26 mg, 0.14 mmol), 1-hydroxybenzotriazole (17 mg, 0.13 mmol), and triethylamine (0.064 ml, 0.46 mmol) in 1 ml of dichloromethane. After stirring overnight at ambient temperature, the reaction mixture was diluted with 30 ml of ethyl acetate, and the organic phase washed sequentially with water, and brine, and then dried over anhydrous magnesium sulfate, filtered and concentrated under vacuum to afford 46 mg of the title compound as a yellow solid. MS (APCI) 492 (M+1)⁺; 490 (M-1)⁻; ¹H NMR (DMSO-d₆) 1.00-1.25 (m, 6H), 3.20-3.35 (m, 2H), 3.40-3.55 (m, 2H), 7.55-7.77 (m, 9H), 7.97 (d, 1H, J = 8.9 Hz), 8.31 (d, 1H, J = 2.3 Hz), 8.40 (d, 1H, J = 1.7 Hz), 8.79 (d, 1H, J = 2.3 Hz), 10.80 (s, 1H).

### Example 41

### 4'-Trifluoromethyl-biphenyl-2-carboxylic acid [3-(piperidine-1-carbonyl)-quinolin-7-yl]-amide

7-[(4'-Trifluoromethyl-biphenyl-2-carbonyl)-amino]-quinoline-3-carboxylic acid (50 mg, 0.11 mmol) was combined with piperidine (10 mg, 0.11 mmol), 1-(3-dimethylaminopropyl)-3-ethylcarbodiimide hydrochloride (26 mg, 0.14 mmol), 1hydroxybenzotriazole (17 mg, 0.13 mmol), and triethylamine (0.064 ml, 0.46 mmol) in 1 ml of dichloromethane. After stirring overnight at ambient temperature, the reaction mixture was concentrated, the residue was suspended in water, and the solid was collected by vacuum filtration to afford 45 mg of the title compound as a colorless solid. MS(APCI) 504 (M+1)⁺; 502 (M-1)⁻; ¹H NMR (DMSO-d₆) 1.40-1.70 (m, 6H), 3.20-3.40 (m, 2H), 3.50-3.70 (m, 2H), 7.55-7.76 (m, 9H), 7.96 (d, 1H, J = 8.9 Hz), 8.31 (d, 1H, J = 2.0 Hz), 8.40 (d, 1H, J = 1.7 Hz), 8.81 (d, 1H, J = 2.3 Hz), 10.80 (s, 1H).

Another preferred group of apo B-secretion/MTP inhibitors useful in the methods of the invention comprises those compounds selected from the group consisting of:
the compound BMS-197636, also known as 9-[4-[4-(2,3-dihydro-1-oxo-1H-isoindol-2-yl)-1-piperidinyl]butyl]-N-propyl-9H-fluorene-9-carboxamide, and the pharmaceutically acceptable salts thereof;
the compound BMS 200150, also known as 2-[1-(3,3-diphenylpropyl)-4-piperidinyl]-2,3-dihydro-1H-isoindol-1-one, and the pharmaceutically acceptable salts thereof;
the compound BMS 201038, also known as 9-[4-(4-[2-(4-trifluoromethylphenyl)benzoylamino]piperidin-1-yl)butyl]-N-2,2,2-trifluoroethyl)-9H-fluorene-9-carboxamide, and the pharmaceutically acceptable salts thereof;
9-{4-[4-(2-benzothiazol-2-yl-benzoylamino)-piperidin-1-yl]-butyl}-9H-fluorene-9-carboxylic acid-(2,2,2-trifluoroethyl)-amide; and the pharmaceutically acceptable salts thereof;
[11a-R]-8-[(4--cyanophenyl)methoxy]-2-cyclopentyl-7-(prop-2-enyl)-2,3,11,11a-tetrahydro-6H-pyrazino[1,2b]isoquinoline-1,4-dione;
[11a-R]-cyclopentyl-7-(prop-2-enyl)-8-[(pyridin-2-yl)methoxy]-2,3,11,11a-tetrahydro-6H-pyrazino[1,2b]-isoquinoline-1,4-dione;
2-cyclopentyl-2-[4-(2,4-dimethyl-pyrido[2,3b]indol-9-ylmethyl)-phenyl]-N-(2-hydroxy-1-phenyl-ethyl)-acetamide; and
2-cyclopentyl-N-(2-hydroxy-1-phenyl-ethyl)-2-[4-(quinolin-2-ylmethoxy)-phenyl]-acetamide.

The compound 9-[4-[4-(2,3-dihydro-1-oxo-1H-isoindol-2-yl)-1-piperidinyl]butyl]-N-propyl-9H-fluorene-9-carboxamide, and the pharmaceutically acceptable salts thereof, may be prepared as disclosed in PCT International Application Publication No. WO 96/26205, the disclosure of which is incorporated herein by reference.

The compound 2-[1-(3,3-diphenylpropyl)-4-piperidinyl]-2,3-dihydro-1H-isoindol-1-one, and the pharmaceutically acceptable salts thereof, may be prepared as disclosed in European Patent Application No. EP 0 643 057, the disclosure of which is also incorporated herein by reference.

The compounds 9-[4-(4-[2-(4-trifluoromethylphenyl)benzoylamino]piperidin-1-yl)butyl]-N-2,2,2-trifluoroethyl)-9H-fluorene-9-carboxamide and 9-{4-[4-(2-benzothiazol-2-yl-benzoylamino)-piperidin-1-yl]-butyl}-9H-fluorene-9-carboxylic acid(2,2,2-trifluoroethyl)-amide, and the pharmaceutically acceptable salts thereof, may be prepared as disclosed in U.S. Pat. No. 5,739,135.

The compounds [11a-R]-8-[(4-cyanophenyl)methoxy]-2-cyclopentyl-7-(prop-2-enyl)-2,3,11,11a-tetrahydro-6H-pyrazino[1,2b]isoquinoline-1,4-dione and [11a-R]-cyclopentyl-7-(prop-2-enyl)-8-[(pyridin-2-yl)methoxy]-2,3,11,11a-tetrahydro-6H-pyrazino[1,2b]-isoquinoline-1,4-dione may be prepared as disclosed in PCT International Application Publication No. WO 98/16526.

The compound 2-cyclopentyl-2-[4-(2,4-dimethyl-pyrido[2,3b]indol-9-ylmethyl)-phenyl]-N-(2-hydroxy-1-phenyl-ethyl)-acetamide may be prepared as disclosed in U.S. Pat. No. 5,684,014.

The compound 2-cyclopentyl-N-(2-hydroxy-1-phenyl-ethyl)-2-[4-(quinolin-2-ylmethoxy)-phenyl]-acetamide may be prepared as disclosed in U.S. Pat. No. 5,646,162.

The disclosures of the aforementioned U.S. Pat. Nos. are incorporated herein by reference in their entirety.

The dosage of the apo B secretion/MTP inhibitor to be administered in accordance with the methods of the invention will generally be dependent upon a number of factors including the health and species of the subject being treated, the extent of treatment desired, the nature and kind of concurrent therapy, if any, and the frequency of treatment and nature of the effect desired. In general, apo B-secretion/MTP inhibitors have been reported with representative dosage ranges being from about 0.01 mg/kg/day to about 15.0 mg/kg/day. Generally preferable dosages range from about 0.1 mg/kg/day to about 1.0 mg/kg/day. However, some variability in the general dosage range may be required depending upon the age, weight, and species of the patient, the intended route of administration, and the like.

According to the methods of the invention, the apo B secretion/MTP inhibitor, a hydrate, stereoisomer, or prodrug thereof, or a pharmaceutically acceptable salt of the inhibitor, hydrate, stereoisomer, or prodrug is administered to the subject in need of treatment therewith prior to, or during, a somnolent period. It is generally preferred that such administration be oral, however, if the subject being treated is unable to swallow, such as when the inhibitor is administered during the somnolent period, or oral absorption is otherwise impaired or undesirable, parenteral or transdermal administration will be appropriate. In an especially preferred method of the invention, the inhibitor is administered orally to the subject being treated immediately prior to bedtime.

According to the methods of the invention, the apo B secretion/MTP inhibitor, a hydrate, stereoisomer, or prodrug thereof, or a pharmaceutically acceptable salt of the inhibitor, hydrate, stereoisomer, or prodrug is preferably administered in the form of a pharmaceutical composition comprising a pharmaceutically acceptable carrier, vehicle, or diluent. Accordingly, the apo B secretion/MTP inhibitor, a hydrate, stereoisomer, or prodrug thereof, or a pharmaceutically acceptable salt of the inhibitor, hydrate, stereoisomer, or prodrug of this invention, can be administered in any conventional oral, parenteral, or transdermal dosage form.

Suitable pharmaceutically-acceptable carriers include inert solid fillers or diluents and sterile aqueous or organic solutions. According to the methods of the invention, the apo B secretion/MTP inhibitor, a hydrate, stereoisomer, or prodrug thereof, or a pharmaceutically acceptable salt of the inhibitor, hydrate, stereoisomer, or prodrug will be present in such pharmaceutical compositions in amounts sufficient to provide the desired dosage amount in the ranges described hereinabove. Thus, for oral administration, the compounds can be combined with a suitable solid or liquid carrier, vehicle or diluent to form capsules, tablets, powders, syrups, solutions, suspensions and the like. The pharmaceutical compositions may, if desired, contain additional components such as flavorants, sweeteners, excipients and the like.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

The pharmaceutical compositions of the invention may also be administered parenterally. For parenteral administration the pharmaceutical compositions can be combined with sterile aqueous or organic media to form injectable solutions or suspensions. Solutions or suspensions of these pharmaceutical compositions can be prepared in water suitably mixed with a surfactant such as hydroxypropylcellulose. Dispersions can also be prepared in sesame or peanut oil, ethanol, water, polyol (e.g., glycerol, propylene glycol and liquid polyethylene glycol), suitable mixtures thereof, vegetable oils, N-methyl glucamine, polyvinylpyrrolidone and mixtures thereof in oils as well as aqueous solutions of water-soluble pharmaceutically acceptable salts of the compounds. Under ordinary conditions of storage and use, these preparations contain a preservative to prevent the growth of microorganisms. The injectable solutions prepared in this manner can then be administered intravenously, intraperitoneally, subcutaneously, or intramuscularly, with intramuscular administration being the preferred parenteral route in humans. Solutions prepared for intravenous administration are preferably rendered isotonic prior to usage.

The pharmaceutical forms suitable for injectable use include sterile aqueous solutions or dispersions and sterile powders for the preparation of sterile injectable solutions or dispersions. In all cases, the form must be sterile and must be fluid to the extent that easy syringability exists. It must be stable under the conditions of manufacture and storage and must be preserved against contamination by microorganisms such as bacteria and fungi.

The pharmaceutical compositions may also be administered transdermally. Suitable formulations for transdermal application include an amount of an apo B secretion/MTP inhibitor, a hydrate, stereoisomer, or prodrug thereof, or a pharmaceutically acceptable salt of the inhibitor, hydrate, stereoisomer, or prodrug, or a pharmaceutical composition thereof, with a suitable transdermal carrier. Preferred transdermal carriers include absorbable pharmacologically acceptable solvents to promote and assist passage through the skin of the subject being treated. Characteristically, transdermal devices comprise the form of a bandage having a backing member, a reservoir containing the compound, optionally with carriers, optionally a rate-controlling barrier to deliver the compound to the skin of the subject being treated at a controlled and predetermined rate over a prolonged period of time and means to secure the device to the skin of the subject being treated.

Methods of preparing the various pharmaceutical compositions with a desired amount of an active ingredient are known, or will be apparent in light of this disclosure, to one of ordinary skill in the art. See, for example, Remington's Pharmaceutical Sciences, Mack Publishing Company, Easton, PA, 18th Edition (1990).

The methods of the instant invention also have utility in the treatment of companion animals, such as dogs and cats. The administration of the compounds or pharmaceutical compositions according to the methods of the invention may be effected orally, parenterally or transdermally. An amount of a compound or pharmaceutical composition of the invention is administered such that an effective dose is received as set forth hereinabove.

Conveniently, the medicament can be carried in the drinking water such that a therapeutic dosage of the agent is ingested with the daily water supply. The agents can be directly metered into drinking water, preferably in the form of a liquid, water-soluble concentrate, such as an aqeuous solution of a water-soluble salt.

For purposes of alternative convenience, the active ingredient can also be added directly to the companion animal's feed, as such, or in the form of an animal feed supplement, also referred to as a premix or concentrate. A premix or concentrate of the therapeutic agent in a carrier is more commonly employed for the inclusion of the agent in the feed. Suitable carriers are liquid or solid, as desired, such as water, various meals such as alfalfa meal, soybean meal, cottonseed oil meal, linseed oil meal, corncob meal and corn meal, molasses, urea, bone meal, and various mineral mixes. A particularly effective carrier is the respective animal feed itself, i.e., a small portion of such feed. The carrier facilitates uniform distribution of the active materials in the finished feed with which the premix is blended. It is important that the compounds be thoroughly blended into the premix and, subsequently, the feed. In this respect, the agents may be dispersed or dissolved in a suitable oily vehicle such as soybean oil, corn oil, cottonseed oil, and the like, or in a volatile organic solvent and then blended with the carrier. It will be appreciated that the proportions of active materials in the concentrate are capable of wide variation since the amount of agent in the finished feed may be adjusted by blending the appropriate proportion of premix with the feed to obtain a desired level of the therapeutic agent.

High potency concentrates may be blended by the feed manufacturer with a proteinaceous carrier such as soybean oil meal and other meals, as described above, to produce concentrated supplements which are suitable for direct feeding to animals. In such instances, the animals are permitted to consume the usual diet. Alternatively, such concentrated supplements may be added directly to the feed to produce a nutritionally balanced, finished feed containing a therapeutically effective level of a compound according to this invention. The mixtures are thoroughly blended by standard procedures, such as in a twin shell blender, to insure homogeniety. If the supplement is used as a top dressing for the feed, it likewise helps to insure uniformity of distribution of the active ingredient across the top of the dressed feed.

For veterinary uses, both paste and pellet formulations may also be conveniently employed. Paste formulations can be prepared readily by dispersing the active compound in a pharmaceutically acceptable oil such as peanut oil, sesame oil, corn oil, and the like. Similarly, pellets containing an effective amount of the compound of the instant invention can be prepared by admixing the compound of this invention with a suitable diluent such as carbowax, carnuba wax, and the like, and a lubricant, such as magnesium or calcium stearate, can be employed to improve the pelleting process.

### EXPERIMENTAL

The general ability of a compound to inhibit the secretion of apo B and/or inhibit MTP can be determined using the following cell based assay, which measures the secretion of apo B in HepG2 cells.

HepG2 cells (ATCC, HB-8065, Manassas, VA) are grown in Dulbecco's Modified Eagles Medium plus 10% fetal bovine serum (Growth medium; Gibco, Grand Island, NY) in 96 well culture plates in a humidified atmosphere containing 5% carbon dioxide until they are approximately 70% confluent. Test compounds are dissolved at 10-20 mM in dimethyl sulfoxide, which is then diluted to 1 µM in growth medium. Serial 1:1 dilutions of this stock are made in growth medium and 100µl of each are added to separate wells of a 96-well culture plate containing HepG2 cells. Twenty four hours later, growth medium is collected and assayed by specific enzyme-linked immunosorbent assay (ELISA) for apo B, and as a control, apo Al concentrations. Inhibitors are identified as compounds that decrease apo B secretion into the medium without affecting the secretion of apo Al. The ELISA for apo B is performed as follows. Monoclonal antibody against human apo B (Chemicon, Temecula, CA) is diluted to 5 µg/ml in phosphate buffered saline/azide (PBS + 0.02% Na azide) and 100µL are added to each well of a 96-well plate (NUNC Maxisorb, Rochester, NY). After an overnight incubation at room temperature, the antibody solution is removed and wells are washed three times with phosphate buffered saline (PBS)/azide. Non-specific sites on the plastic are blocked by incubating wells for 1-3 hours in a solution of 1 % (w/v) bovine serum albumin (BSA) made in PBS/azide. 100 µL of various dilutions of growth medium from the HepG2 cells or apo B standards (made in 0.004% Tween 20/1% BSA in PBS/azide) are added to each well and incubated for 18 hours. Wells are aspirated and washed three times (0.1 % Tween 20 in PBS) prior to adding 100µL of a 1/1000 dilution of the secondary antibody, goat anti-human apo B (Chemicon). After 3 hours incubation at room temperature, this solution is aspirated and the wells are again washed 3 times as above. 100µl of a 1:1600 dilution (in PBS/1% BSA/2 mM MgCl₂) of rabbit anti-goat IgG conjugated to alkaline phosphatase (Sigma, Milwaukee, Wl) are then added to each well and incubated for 1 hour at room temperature. After aspirating, the wells are washed 4 times as above and 100µl of 1mg/ml p-nitrophenylphosphate (pNPP; Sigma) in 25 mM sodium bicarbonate/2mM MgCl₂, pH 9.5, are added to each well and incubated for 20-30 minutes and then the reaction is terminated by the addition of 50µL of 0.2N NaOH. Absorbance of each well is read at 405 nm and the background at 650 nm is subtracted. Apo B concentration is calculated from a standard curve constructed from purified LDL standards that are run in parallel in the same assay. Apo Al is measured in an analogous manner except that antibodies for apo Al (Chemicon) are used in place of the antibodies for apo B and antigen incubation is at 37°C instead of room temperature.

The direct inhibition of MTP activity by a compound can be quantitated by observing the inhibition of radiolabeled triglyceride from the donor vesicles to acceptor vesicles in the presence of soluble human MTP. The procedures for preparing MTP are based on the method of Wetterau, et al., Biochem. Biophys. Acta, 875, 610 (1986). Briefly, human liver chunks, frozen at -80°C, are thawed on ice, minced, and rinsed several times with ice cold 0.25M sucrose. All subsequent steps are performed on ice. A 50% homogenate in 0.25 M sucrose is prepared using a Potter-Elvehjem Teflon pestle. The homogenate is diluted 1:1 with 0.25 M sucrose and centrifuged at 10,000 x g for 20 minutes at 4°C. The pellet is resuspended in sucrose and recentrifuged at 10,000 x g for 20 minutes. The supernatants are combined and the microsomes pelleted by centrifugation at 105,000 x g for 75 minutes. The supernatant is decanted and the microsomal pellet is suspended in a minimal volume of 0.25 M sucrose, diluted to 3 ml per gram starting liver weight with 0.15M Tris-HCI, pH 8.0. This suspension is divided into 12 fractions, and centrifuged at 105,000 x g for 75 minutes. The supernatants are discarded and the microsomal pellets are stored frozen at -80°C until needed. For preparation of MTP prior to performing the assay, a thawed pellet is suspended in 12 ml of cold 50 mM Tris-HCI, 50 mM KCI, 5 mM MgCl₂, pH 7.4 and 1.2 ml of a 0.54% deoxycholate (pH 7.4) solution is added slowly with mixing to disrupt the microsomal membrane. After 30 minutes incubation on ice with gentle mixing, the suspension is centrifuged at 105,000 x g for 75 minutes. The supernatant containing the soluble MTP protein is dialyzed for 2-3 days with 4 changes of assay buffer (150 mM Tris-HCI, 40 mM NaCI, 1 mM EDTA, 0.02% NaN3, pH 7.4). The human liver MTP is stored at 4°C and diluted 1:5 with assay buffer just before use. MTP preparations show no notable loss of transfer activity with storage up to 30 days.

Liposomes are prepared under nitrogen by room temperature bath sonication of a dispersion of 400 µM egg phosphatidylcholine (PC), 75 µM bovine heart cardiolipin, and 0.82µM [¹⁴C]-triolein (110Ci/mol) [New England Nuclear, Boston, MA] in assay buffer. The lipids in chloroform are added in the proper amounts and dried under a nitrogen stream before hydrating with assay buffer. Acceptor liposomes are prepared under nitrogen by room temperature bath sonication of a dispersion of 1.2 mM PC, 2.3 µM triolein and 30 pM [³H]-PC (50Ci/mol) in assay buffer. The donor and acceptor liposomes are centrifuged at 160,000 x g for 2 hours at 7°C. The top 80% of the supernatant containing small unilamellar liposomes are carefully removed and stored at 4°C until used for transfer assays.

MTP activity is measured using a transfer assay that is initiated by mixing donor and acceptor vesicles together with the soluble MTP and test compound. To 100 µl of either a 5% BSA (control) or 5% BSA containing the test compound are added 500 µl assay buffer, 100 µl donor liposomes and 100µl of diluted MTP protein. After incubation at 37°C for 45 minutes, triglyceride transfer is terminated by adding 500 µL of a 50% (w/v) diethylaminoethyl (DEAE) cellulose suspension in assay buffer. Following 4 minutes of agitation, the donor liposomes, bound to DEAE cellulose are selectively sedimented by low speed centrifugation. An aliquot of the supernatant containing the acceptor liposomes is counted and the ³H and ¹⁴C counts are used to calculate the percent recovery of acceptor liposomes and the percent triglyceride transfer using first order kinetics. Inhibition of triglyceride transfer by test compound is manifest as a decrease in ¹⁴C radioactivity compared to controls where no test compound is present.

The activity of a test compound as an MTP inhbitor can also be determined *in vivo* according to the following procedure.

Male mice (20-30 g, various strains) are dosed by oral gavage (0.25 mL/25 g body weight) with a test compound suspended in an aqueous 0.5% methyl cellulose solution. Compound solutions are dosed either multiple times, over several days, or, alternatively, once 90 minutes before mice are euthanized and blood is collected for preparation of serum. The serum is assayed for triglyceride concentration by a commercial enzymatic assay (Triglyceride G: Wako Fine Chemicals, Osaka, Japan). MTP inhibitors are identified by their ability to lower serum triglycerides as compared to control mice dosed with vehicle.

### HUMAN PHARMACOLOGICAL EVALUATION

The utility of administering an apo B-secretion/MTP inhibitor according to the methods of the invention is exemplified by the improvement in efficacy of the test compound 4'-trifluoromethyl-biphenyl-2-carboxylic acid-(2-(1H-(1,2,4-triazol-3-ylmethyl)-1,2,3,4-tetrahydroisoquinolin-6-yl)-amide when administered according to the following human clinical protocol. The various end-points quantitated to demonstrate the improvement in efficacy are detailed hereinbelow.

A three-group, randomized, double-blind, placebo-controlled, parallel group study, using healthy male subjects between 18 and 45 years of age inclusive, was conducted to determine, *inter alia,* the lipid level profiles of multiple doses of the test compound administered once daily in the morning as compared to the same dose administered in the evening at bedtime.

The first subject group was given a 10 mg dose of the test compound and the second subject group was given a 30 mg dose of the test compound. The 30 mg subject group was divided into two sub-groups, one of which was administered the test compound in the morning and the other administered the test compound in the evening at bedtime. Each sub-group was made up of 10 subjects, 8 of which were randomized to receive the test compound and 2 of which received placebo. On Day 1, one sub-group received either the test compound or placebo at approximately 7:00 am. They subsequently received test compound or placebo at approximately 7:00 am daily from Days 4-17. The other sub-group received either the test compound or placebo at approximately 10:00 pm (bedtime) on Day 1. From Days 4-17 they also received either the test compound or placebo at approximately 10:00 pm.

On Days 1 and 17, subjects were fasted for at least 8 hours prior to the morning dosing, and continued to fast for 4 hours after test compound administration, for the dose groups evaluated in the morning. A standard meal was then served. On other days, breakfast was served approximately 2 hours after the morning dosing. On Days 4 and 17, subjects were required to refrain from lying down, except for during the monitoring of vital signs and safety assessments, for at least 4 hours after test compound administration in order to standardize experimental conditions. Access to drinking water was permitted *ad libitum.* For the dose groups evaluated in the evening, the test compound was administered at least 4 hours after the last major meal.

### PHARMACODYNAMIC PROFILINGS

The pharmacodynamic profiles of the test compound were evaluated following administration of the test compound. The pharmacodynamic end-points quantitated included total plasma cholesterol and LDL-cholesterol.

On Days 1 and 17 of the study, blood sufficient to provide approximately 3 mL plasma was collected at the following times: pre-dose, at 1, 2, 4, 6, 8, 10, and 24 hours after test compound administration. In addition, on Days 6, 7, 8,10,12,14, and 16, pre-dose blood samples (am and pm, where indicated) was obtained for measurement of blood lipids. Blood samples were collected into tubes containing the anti-coagulant EDTA. The plasma was separated from whole blood in a refrigerated centrifuge, transferred into labeled tubes and stored frozen at -20° C until quantitation of the various end-points was performed.

Preparative ultracentrifugation was employed using NCCLS Guidelines (Manual of Laboratory Operations, Lipid Research Clinics Program, I: Lipid and Lipoprotein Analysis. National Heart, Lung, and Blood Institute, National Institutes of Health, Bethesda, Maryland, 1974, U.S. Government Printing Office (NIH) 75628), and the methodologies set forth in Thompson, G.R., A Handbook of Hyperlipidaemia, (Current Science Ltd., London), 43-49, (1989), to separate the various lipoprotein classes. Quantitation of total plasma cholesterol and LDL-cholesterol was performed on a Hitachi 747 Chemical Analyzer (Boehringer-Mannheim, Indianapolis, IN) employing SAS 6.12 software (Hewlett-Packard Instrument Company, Cupertino, CA).

The mean percent decreases, corrected for placebo, in total serum cholesterol (mg/dL) and LDL-cholesterol (mg/dL) at Day 17, attributable to the administration of the test compound are summarized hereinbelow. Analyses of the pertinent end-points indicated significantly enhanced dose-related mean percent decreases in total serum cholesterol and LDL-cholesterol attributable to the administration of the test compound when the compound was administered in the evening, i.e. prior to the somnolent period of the subject being treated, as opposed to administration of the test compound in the morning.

The magnitude of the mean percent decrease in total serum cholesterol levels was greatest for the group administered with the test compound at bed-time, with a mean percent decrease in total cholesterol from baseline of -40% on Day 17. The mean percent decrease in total cholesterol from baseline following administration of test compound in the morning was approximately -21%, which represents an improvement in drug efficacy of 19% for evening, as compared to morning, administration.

The magnitude of the mean percent decrease in LDL-cholesterol levels was also greatest for the group administered with the test compound at bed-time, with a mean percent decrease in LDL-cholesterol from baseline of -80% on Day 17. The mean percent decrease in LDL-cholesterol from baseline following administration of test compound in the morning was approximately -32%, which represents an improvement in drug efficacy of 48% for evening, as compared to morning, administration.

## Claims

1. The use of an apolipoprotein B-secretion (apo B)/microsomal triglyceride transfer protein (MTP) inhibitor in the preparation of a medicament for the reduction of total serum cholesterol and LDL-cholesterol which is adminstered to the subject prior to or during a somnolent period.

2. The use as claimed in claim 1 wherein said apolipoprotein B-secretion/microsomal triglyceride transfer protein inhibitor is selected from:
(i) a compound of formula (I) 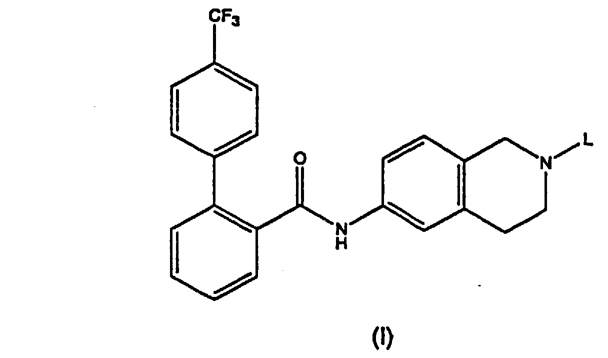 the stereoisomers, and hydrates thereof, and the pharmaceutically acceptable salts of said compounds, stereoisomers and hydrates, wherein L represents:
X-Y-Z, wherein:
X is.a.moiety selected from the group consisting of CH₂, CO, CS, or SQ₂;
Y is a moiety selected from the group consisting of a direct link, aliphatic hydrocarbylene radicals having up to 20 carbon atoms, which radical may be monosubstituted by hydroxy, (C₁-C₁₀)alkoxy, (C₁-C₁₀)acyl, (C₁-C₁₀)acyloxy, or (C₆-C₁₀)aryl, NH, and O, provided that if X is CH₂, Y is a direct link; and
Z is a moiety selected from the group consisting of:
(1) hydrogen, halogen, cyano,
(2) hydroxy, (C₁-C₁₀)alkoxy, (C₁-C₁₀)alkylthio, (C₁-C₁₀)acyl, thiophenylcarbonyl, (C₁-C₁₀)alkoxycarbonyl,
(3) (C₁-C₁₀)alkylamino, di(C₁-C₁₀)alkylamino, (C₆-C₁₀)aryl(C₁-C₁₀)alkylamino, provided that Y is not O or NH,
(4) unsubstituted vinyl, (C₆-C₁₀)aryl, (C₃-C₈)cycloalkyl and fused benz derivatives thereof, (C₇-C₁₀)Polycycloalkyl, (C₄-C₈)cycloalkenyl, (C₇-C₁₀)polycycloalkenyl,
(5) (C₆-C₁₀)aryloxy, (C₆-C₁₀)arylthio, (C₆-C₁₀)aryl(C₁-C₁₀)alkoxy, (C₆-C₁₀)aryl(C₁-C₁₀)alkylthio, (C₃-C₈)cycloalkyloxy, (C₄-C₈)cycloalkenyloxy,
(6) heterocyclyl selected from the group consisting of monocyclic radicals and fused polycyclic radicals, wherein said radicals contain a total of from 5 to 14 ring atoms, wherein said radicals contain a total of from 1 to 4 ring heteroatoms independently selected from oxygen, nitrogen, and sulfur, and wherein the individual rings of said radicals may be independently saturated, partially unsaturated, or aromatic, provided that if X is CH₂, Z is H or is selected from groups (4) and (6),
wherein, when Z contains one or more rings, said rings may each independently bear 0 to 4 substituents independently selected from halo, hydroxy, cyano, nitro, oxo, thioxo, aminosulfonyl, phenyl, phenoxy, phenylthio, halophenylthio, benzyl, benzyloxy, (C₁-C₁₀)alkyl, (C₁-C₁₀)alkoxy, (C₁-C₁₀)alkoxycarbonyl, (C₁-C₁₀)alkylthio, (C₁-C₁₀)alkylamino, (C₁-C₁₀)alkylaminocarbonyl, di(C₁-C₁₀)alkylamino, di(C₁-C₁₀)alkylaminocarbonyl, di(C₁-C₁₀)alkylamino(C₁-C₁₀)alkoxy, (C₁-C₃)perfluoroalkyl, (C₁-C₃)perfluoroalkoxy, (C₁-C₁₀)acyl, (C₁-C₁₀)acyloxy, (C₁-C₁₀)acyloxy(C₁-C₁₀)alkyl, and pyrrolidinyl; or
G, wherein G is selected from the group consisting of:
(a) a phenyl or heterocyclic ring wherein said heterocyclic ring contains a total of from 3 to 14 ring atoms, wherein said heterocyclic ring incorporates a total of from 1 to 4 ring heteroatoms selected independently from oxygen, nitrogen, and sulfur, wherein the individual rings of said heterocyclic ring may be independently saturated, partially saturated or aromatic, and wherein each of said phenyl or heterocyclic rings may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acyloxy, (C₁-C₆)acylamino and (C₁-C₆)perfluoroacylamino;
(b) -CH₂CN,
(c) 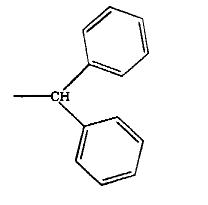
(d) (C₂-C₁₂)alkyl or (C₂-C₁₂)perfluoroalkyl wherein each of said (C₂-C₁₂)alkyl and (C₂-C₁₂)perfluoroalkyl is substituted optionally with from 1-3 substituents selected independently from:
(1) phenyl, halogen, nitro, cyano, hydroxy, -NR¹R², -OCOR³, (C₁-C₄)alkoxy, (C₁-C₄)perfluoroalkoxy, (C₁-C₄)thioalkoxy or (C₁-C₄)perfluorothioalkoxy,
where R¹ and R² in the definition of -NR¹R² are each selected independently from hydrogen, formyl, phenyl, benzyl, benzoyl, (C₃-C₈)cycloalkyl, (C₃-C₈)cycloalkenyl, (C₁-C₄)alkyl, (C₁-C₄)perfluoroalkyl, (C₁-C₁₀)alkoxycarbonyl, (C₁-C₆)acyl, (C₁-C₆)perfluoroacyl, aminocarbonyl, (C₁-C₁₀)alkylaminocarbonyl, di(C₁-C₁₀)alkylaminocarbonyl, aminosulfonyl, (C₁-C₄)alkylaminosulfonyl, di(C₁-C₄)alkylaminosulfonyl, (C₁-C₄)perfluoroalkylaminosulfonyl, di(C₁-C₄)perfluoroalkylaminosulfonyl, (C₁-C₄)alkylsulfonyl, and (C₁-C₄)perfluoroalkylsulfonyl,
or where R¹ and R², taken together with the nitrogen atom to which they are attached, form a saturated, partially-saturated or aromatic heterocyclic ring, wherein said heterocyclic ring contains a total of from 3 to 14 ring atoms and incorporates optionally an additional 1 to 4 ring heteroatoms selected independently from oxygen, nitrogen and sulfur, wherein said heterocyclic ring may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acylamino, and (C₁-C₁₀)acyloxy,
where R³ in the definition of -OCOR³ is selected from -NR¹R², phenyl, (C₁-C₁₀)alkyl, (C₁-C₄)perfluoroalkyl, (C₁-C₆)alkoxy and (C₁-C₆)perfluoroalkoxy,
(2) (C₃-C₈)cycloalkyl or (C₃-C₈)cycloalkenyl wherein each of said (C₃-C₈)cycloalkyl and (C₃-C₈)cycloalkenyl may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acylamino, (C₁-C₁₀)perfluoroacylamino, (C₁-C₁₀)acyloxy, and
(3) a saturated, partially-saturated or aromatic heterocyclic ring containing a total of from 3 to 14 ring atoms, wherein said heterocyclic ring incorporates a total of from 1 to 4 ring heteroatoms selected independently from oxygen, nitrogen and sulfur, wherein said heterocyclic ring may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acylamino, (C₁-C₁₀)perfluoroacylamino, (C₁-C₁₀)acyloxy;
(e) (C₃-C₈)cycloalkyl or (C₃-C₈)cycloalkenyl wherein each of said (C₃- C₈)cycloalkyl and (C₃-C₈)cycloalkenyl may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acylamino, (C₁-C₁₀)perfluoroacylamino, (C₁-C₁₀)acyloxy; and
(f) -(CH₂)ₙCOR⁴, where R⁴ in the definition of -(CH₂)ₙCOR⁴ is selected from hydroxy, phenyl, -NR¹R², (C₁-C₄)alkyl, (C₁-C₄)perfluoroalkyl, (C₁-C₄)alkoxy, (C₁-C₄)perfluoroalkoxy, (C₃-C₈)cycloalkyl, and (C₃-C₈)cycloalkenyl, where n is an integer from 1 to 4;
(ii) a compound of formula (Ia) 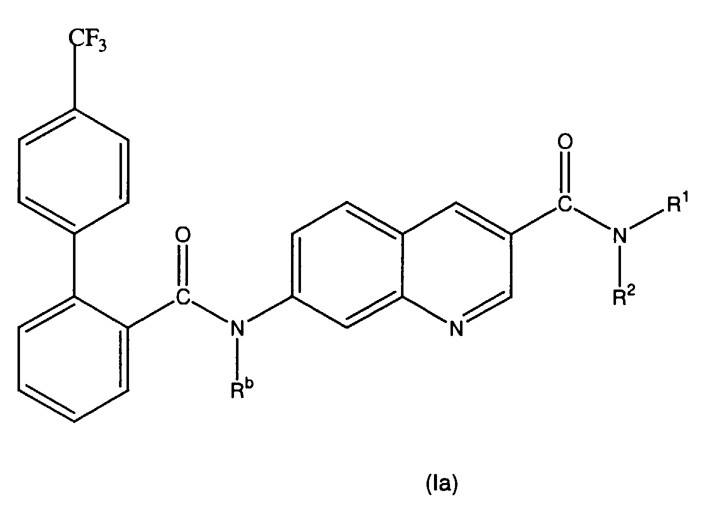 the stereoisomers and prodrugs thereof, and the pharmaceutically acceptable salts of said compounds, stereoisomers, and prodrugs, wherein
each R^{a} and R^{b} is independently hydrogen or C₁-C₈alkyl;
each n is independently 0, 1, 2 or 3;
each X is independently aryl, substituted aryl, heteroaryl, substituted heteroaryl, cycloalkyl, substituted cycloalkyl, heterocycloalkyl, or substitited heterocycloalkyl;
R¹ is hydrogen or C₁-C₈alkyl; and
R² is hydrogen, -(CR^{a}R^{a})ₙ-X, C₁-C₈alkyl, C₁-C₈substituted alkyl, 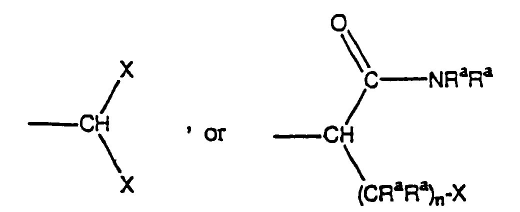
or R¹ and R² together with the nitrogen atom to which they are bonded form a 3 to 7 membered heterocycloalkyl ring comprising from 1 to 3 heteroatoms; and
(III) a compound selected from the group consisting of:
9-[4-[4-(2,3-dihydro-1-oxo-1H-isoindol-2-yl)-1-piperidinyl]butyl]-N-propyl-9H-fluorene-9-carboxamide, and the pharmaceutically acceptable salts thereof;
2-[1-(3,3-diphenylpropyl)-4-piperidinyl]-2,3-dihydro-1H-isoindol-1-one, and the pharmaceutically acceptable salts thereof;
9-[4-(4-[2-4-bifluoromethylphenyl)benzoylamino]piperidin-1-yl)butyl]-N-2,2,2-trifluoroethyl)-9H-fluorene-9-carboxamide, and the pharmaceutically acceptable salts thereof;
9-[4-[4-(2-benzothiazol-2-yl-benzoylamino)-piperidin-1-yl]-butyl]-9H-fluorene-9-carboxylic acid-{2,2,2-trifluoroethyl)-amide, and the pharmaceutically acceptable salts thereof;
[11a-R]-8-[(4-cyanophenyl)methoxy]-2-cyclopentyl-7-(prop-2-enyl)-2,3,11,11a-tetrahydro-6H-pyrazino[1,2b]isoquinoline-1,4-dione;
[11a-R]-cyclopentyl-7-(prop-2-enyl)-8[(pyridin-2-yl)methoxy]-2,3,11,11a-tetrahydro-6H-pyrazino-[1,2b]-isoquinoline-1,4-dione;
2-cyclopentyl-2-[4-(2,4-dimethyl-pyrido[2,3b]indol-9-ylmethyl)-phenyl]-N-(2-hydroxy-1-phenyl-ethyl)-acetamide; and
2-cyclopentyl-N-(2-hydroxy-1-phenyl-ethyl)-2-[4-(quinoin-2-ylmethoxy)-phenyl]-acetamide.

3. The use as claimed in claim 2 wherein said inhibitor is a compound of formula (I) 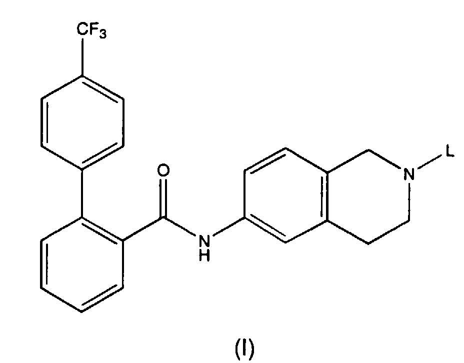 the stereoisomers and hydrates thereof, and the pharmaceutically acceptable salts of said compounds, stereoisomers and hydrates, wherein L represents:
X-Y-Z, wherein:
X is a moiety selected from the group consisting of CH₂, CO, CS, or SO₂;
Y is a moiety selected from the group consisting of a direct link, aliphatic hydrocarbylene radicals having up to 20 carbon atoms, which radical may be monosubstituted by hydroxy, (C₁-C₁₀)alkoxy, (C₁-C₁₀)acyl, (C₁-C₁₀)acyloxy, or (C₆-C₁₀)aryl, NH, and O, provided that if X is CH₂, Y is a direct link; and
Z is a moiety selected from the group consisting of:
(1) hydrogen, halogen, cyano,
(2) hydroxy, (C₁-C₁₀)alkoxy, (C₁-C₁₀)alkylthio, (C₁-C₁₀)acyl, thiophenylcarbonyl, (C₁-C₁₀)alkoxycarbonyl,
(3) (C₁-C₁₀)alkylamino, di(C₁-C₁₀)alkylamino, (C₆-C₁₀)aryl(C₁-C₁₀)alkylamino, provided that Y is not O or NH,
(4) unsubstituted vinyl, (C₆-C₁₀)aryl, (C₃-C₈)cycloalkyl and fused benz derivatives thereof, (C₇C₁₀)polycycloalkyl, (C₄-C₈)cycloalkenyl, (C₇-C₁₀)polycycloalkenyl,
(5) (C₆-C₁₀)aryloxy, (C₆-C₁₀)arylthio, (C₆-C₁₀)aryl(C₁-C₁₀)alkoxy, (C₆-C₁₀)aryl(C₁-C₁₀)alkylthio, (C₃-C₈)cycloalkyloxy, (C₄-C₈)cycloalkenyloxy,
(6) heterocyclyl selected from the group consisting of monocyclic radicals and fused polycyclic radicals, wherein said radicals contain a total of from 5 to 14 ring atoms, wherein said radicals contain a total of from 1 to 4 ring heteroatoms independently selected from oxygen, nitrogen, and sulfur, and wherein the individual rings of said radicals may be independently saturated, partially unsaturated, or aromatic, provided that if X is CH₂, Z is H or is selected from groups (4) and (6),
wherein, when Z contains one or more rings, said rings may each independently bear 0 to 4 substituents independently selected from halo, hydroxy, cyano, nitro, oxo, thioxo, aminosulfonyl, phenyl, phenoxy, phenylthio, halophenylthio, benzyl, benzyloxy, (C₁-C₁₀)alkyl, (C₁-C₁₀)alkoxy, (C₁-C₁₀)alkoxycarbonyl, (C₁-C₁₀)alkylthio, (C₁-C₁₀)alkylamino, (C₁-C₁₀)alkylaminocarbonyl, di(C₁-C₁₀)alkylamino, di(C₁-C₁₀)alkylaminocarbonyl, di(C₁-C₁₀)alkylamino(C₁-C₁₀)alkoxy, (C₁-C₃)perfluoroalkyl, (C₁-C₃)perfluoroalkoxy, (C₁-C₁₀)acyl, (C₁-C₁₀)acyloxy, (C₁-C₁₀)acyloxy(C₁-C₁₀)alkyl, and pyrrolidinyl; or
G, wherein G is selected from the group consisting of:
(a) a phenyl or heterocyclic ring wherein said heterocyclic ring contains a total of from 3 to 14 ring atoms, wherein said heterocyclic ring incorporates a total of from 1 to 4 ring heteroatoms selected independently from oxygen, nitrogen, and sulfur, wherein the individual rings of said heterocyclic ring may be independently saturated, partially saturated or aromatic, and wherein each of said phenyl or heterocyclic rings may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acyloxy, (C₁-C₆)acylamino and (C₁-C₆)perfluoroacylamino;
(b) -CH₂CN,
(c) 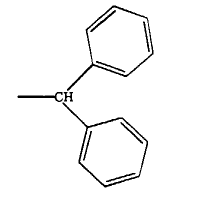
(d) (C₂-C₁₂)alkyl or (C₂-C₁₂)perfluoroalkyl wherein each of said (C₂-C₁₂)alkyl and (C₂-C₁₂)perfluoroalkyl is substituted optionally with from 1-3 substituents selected independently from:
(1) phenyl, halogen, nitro, cyano, hydroxy, -NR¹R², -OCOR³, (C₁-C₄)alkoxy, (C₁-C₄)perfluoroalkoxy, (C₁-C₄)thioalkoxy or (C₁-C₄)perfluorothioalkoxy,
where R¹ and R² in the definition of -NR¹R² are each selected independently from hydrogen, formyl, phenyl, benzyl, benzoyl, (C₃-C₈)cycloalkyl, (C₃-C₈)cycloalkenyl, (C₁-C₄)alkyl, (C₁-C₄)perfluoroalkyl, (C₁-C₁₀)alkoxycarbonyl, (C₁-C₆)acyl, (C₁-C₆)perfluoroacyl, aminocarbonyl, (C₁-C₁₀)alkylaminocarbonyl, di(C₁-C₁₀)alkylaminocarbonyl, aminosulfonyl, (C₁-C₄)alkylaminosulfonyl, di(C₁-C₄)alkylaminosulfonyl, (C₁-C₄)perfluoroalkylaminosulfonyl, di(C₁-C₄)perfluoroalkylaminosulfonyl, (C₁-C₄)alkylsulfonyl, and (C₁-C₄)perfluoroalkylsulfonyl,
or where R¹ and R², taken together with the nitrogen atom to which they are attached, form a saturated, partially-saturated or aromatic heterocyclic ring, wherein said heterocyclic ring contains a total of from 3 to 14 ring atoms and incorporates optionally an additional 1 to 4 ring heteroatoms selected independently from oxygen, nitrogen and sulfur, wherein said heterocyclic ring may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acylamino, and (C₁-C₁₀)acyloxy,
where R³ in the definition of -OCOR³ is selected from -NR¹R², phenyl, (C₁-C₁₀)alkyl, (C₁-C₄)perfluoroalkyl, (C₁-C₆)alkoxy and (C₁-C₆)perfluoroalkoxy,
(2) (C₃-C₈)cycloalkyl or (C₃-C₈)cycloalkenyl wherein each of said (C₃-C₈)cycloalkyl and (C₃-C₈)cycloalkenyl may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C4)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acylamino, (C₁-C₁₀)perfluoroacylamino, (C₁-C₁₀)acyloxy, and
(3) a saturated, partially-saturated or aromatic heterocyclic ring containing a total of from 3 to 14 ring atoms, wherein said heterocyclic ring incorporates a total of from 1 to 4 ring heteroatoms selected independently from oxygen, nitrogen and sulfur, wherein said heterocyclic ring may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkflamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acylamino, (C₁-C₁₀)perfluoroacylamino, (C₁-C₁₀)acyloxy;
(e) (C₃-C₈)cycloalkyl or (C₃-C₈)cycloalkenyl wherein each of said (C₃-C₈)cycloalkyl and (C₃-C₈)cycloalkenyl may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkythio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acylamino, (C₁-C₁₀)perfluoroacylamino, (C₁-C₁₀)acyloxy; and
(f) -(CH₂)ₙCOR₄, where R⁴ in the definition of -(CH₂)ₙCOR⁴ is selected from hydroxy, phenyl, -NR¹R², (C₁-C₄)alkyl, (C₁-C₄)perfluoroalkyl, (C₁-C₄)alkoxy, (C₁-C₄)perfluoroalkoxy, (C₃-C₈)cycloalkyl, and (C₃-C₈)cycloalkenyl, where n is an integer from 1 to 4.

4. The use as claimed in claim 3 wherein said compound of formula (I) is selected from the group consisting of:
4'-trifluoromethyl-biphenyl-2-carboxylic acid-(2-(1H-(1,2,4-triazol-3-ylmethyl)-1,2,3,4-tetrahydroisoquinolin-6-yl)-amide;
4'-trifluoromethyl-biphenyl-2-carboxylic acid-[2-(2-acetylaminoethyl)-1,2,3,4-tetrahydroisoquinolin-6-yl amide;
4'-trifluoromethyl-biphenyl-2-carboxylic acid-[2-(2-methoxyethyl)-1,2,3,4-tetrahydroisoquinlin-6-yl]-amide;
4'-trifluoromethyl-biphenyl-2-carboxylic acid-[2-(2,2-diphenylethyl)-1,2,3,4-tetrahydroisoquinlin-6-yl]-amide;
2-(6-(4'-trifluoromethyl-biphenyl-2-carbonyl)-amino]-3,4-dihydro-1H-isoquinolin-2-yl)-ethyl)-carbamic acid methyl ester;
4'-trifluoromethyl-biphenyl-2-catoxylic acid-[2-(thiophen-2-yl-acetyl)-1,2,3,4-tetrahydroisoquinlin-6-yl]-amide;
4'-trifluoromethyl-biphenyl-2-carboxyic acid-(2-pyridin-2-ylmethyl)-1,2,3,4-tetralhydroisoquinlin-6-yl)-amide; and
6-[(4'-trifluoromethyl-biphenyl-2-cabonyl)-amino]-3,4-dihydro-1H-isoquinoline-2-carboxylic add [(R)-1-phenyl-ethyl]-amide; the stereoisomers and hydrates thereof, and the pharmaceutically acceptable salts of said compounds, stereoisomers, and hydrates.

5. The use as claimed in claim 2 wherein said inhibitor is a compound of formula (la) 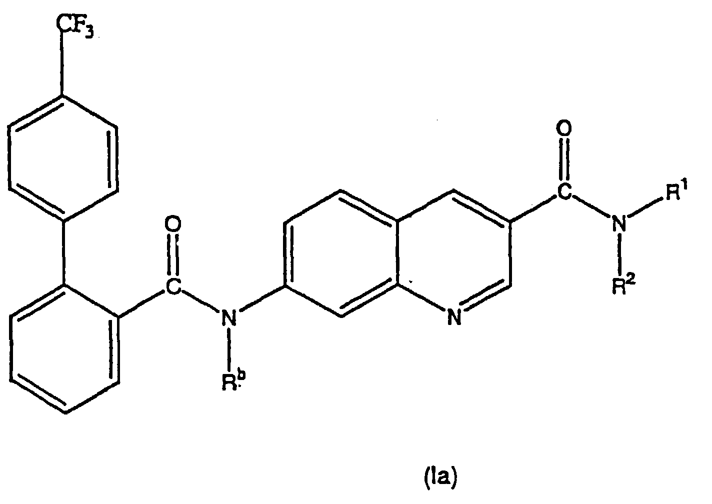
the stereoisomers and prodrugs thereof, and the pharmaceutically acceptable salts of
said compounds, stereoisomers, and prodrugs, wherein
each R^{a} and R^{b} is independently hydrogen or C₁-C₈alkyl;
each n is independently 0, 1, 2 or 3;
each X is independently aryl, substituted aryl, heteroaryl, substituted heteroaryl,
cycloalkyl, substituted cycloalkyl, heterocycloalkyl, or substitited heterocycloalkyl;
R¹ is hydrogen or C₁-C₈alkyl; and
R² is hydrogen, -(CR^{a}R^{a})ₙ-X, C₁-C₈alkyl, C₁-C₈substituted alkyl, 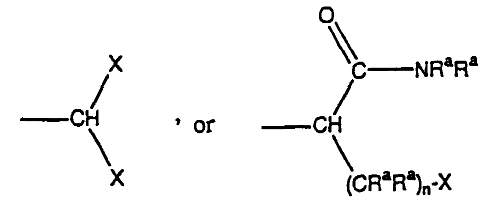
or R¹ and R² together with the nitrogen atom to which they are bonded form a 3 to 7 membered heterocycloalkyl ring comprising from 1 to 3 heteroatoms.

6. The use as claimed in claim 5 wherein said compound of formula (la) is selected from the group consisting of:
7-[(4'-trifluoromethyl-biphenyl-2-carbonyl)-amino]-quinoline-3-carboxylic acid-(diphenylpyridin-2-yl-methyl)-amide;
7-[(4'-triluoromethyl-biphenyl-2-carbonyl)-amino]-quinoline-3-carboxylic acid-(phenylpyridin-2-yl-methyl)-amide;
7-((4'-trifluoromethyl-biphenyl-2-carbonyl)-amino]-quinoline-3-carboxylic acid**(1**-methyl-1-phenyl-ethyl)-amide;
7-[(4'-trifluoromethyl-biphenyl-2-carbonyl)-amino]-quinoline-3-carboxylic acid(1-phenyl-propyl)-amide;
(R)-7-[(4'-trifluoromethyl-biphenyl-2-carbonyl)-amino]-quinoline-3-carboxylic acid-(1-phenyl-ethyl)-amide;
7-[(4'-trifluoromethyl-biphenyl-2-carbonyl)-amino]-quinoline-3-carboxylic acid-(1-pyridin-2-yl-propyl)-amide;
7-[(4'-trifluoromethyl-biphenyl-2-carbonyl)-amino]-quinoline-3-carboxylic acid-(2-pyridin-2-yl-ethyl)-amide;
7-[(4'-trifluoromethyl-biphenyl-2-carbonyl)-amino]-quinoline-3-carboxylic acid-ethylamide;
7-[(4'-trifluoromethyl-biphenyl-2-carbonyl)-amino]-quinoline-3-carboxylic acid-isopropylamide; and
7-[(4'-trifluoromethyl-biphenyl-2-carbonyl)-amino]-quinoline-3-carboxylic acid-diethylamide; the stereoisomers and prodrugs thereof, and the pharmaceutically acceptable salts of said compounds, stereoisomers and prodrugs.

7. The use as claimed in claim 2 wherein said inhibitor is a compound selected from the group consisting of:
9-[4-(4-(2,3-dihydro-1-oxo-1H-isoindol-2-yl)-1-piperidinyl]butyl]-N-propyl-9H-fluorene-9-carboxamide, and the pharmaceutically acceptable salts thereof;
2-[1-(3,3-diphenylpropyl)-4-piperidinyl]-2,3-dihydro-1H-isoindol-1-one, and the pharmaceutically acceptable salts thereof;
9-[4-(4-[2-(4-trifluoromethylphenyl)benzoylamino]piperidin-1-yl)butyq-N-2,2,2-trifluoroethyl)-9H-fluorene-9-carboxamide, and the pharmaceutically acceptable salts thereof;
9-(4-[4-(2-benzothiazol-2-yl-benzoylamino)-piperidin-1-yl]-butyl)-9H-fluorene-9-carboxylic acid-(2,2,2-trifluoroethyl)-amide, and the pharmaceutically acceptable salts thereof;
[11 a-R]-8-[(4-cyanophenyl)methoxy]-2-cyclopentyl-7-(prop-2-enyl)-2,3,11,11a-tetrahydro-6H-pyrazino[1,2b]isoquinoline-1,4-dione;
[11a-R]-Cyclopentyl-7-(prop-2-enyl)-8-[(pyridin-2-yl)methoxy]-2,3,11,11a-tetrahydro-6H-pyrazino[1,2b]-isoquinoline-1,4-dione;
2-cyclopentyl-2-[4-(2,4-dimethyl-pyrido[2,3b]indol-9-ylmethyl)-phenyl]-N-(2-hydroxy-1-phenyl-ethyl)-acetamide; and
2-cyclopentyl-N-(2-hydroxy-1-phenyl-ethyl)-2-[4-(quinolin-2-ylmethoxy)-phenyl]-acetamide.

8. The use as claimed in claim 1 wherein said medicament is intended for oral administration.

9. The use as claimed in claim 1 wherein said medicament is intended for parenteral administration.

10. The use as claimed in claim 1 wherein said medicament is intended for transdermal administration.
